(19) Europäisches Patentamt
European Patent Office
Office européen des brevets

(11) Veröffentlichungsnummer: **0 172 140 B1**

# (12) EUROPÄISCHE PATENTSCHRIFT

(45) Veröffentlichungstag der Patentschrift: **06.09.89**

(51) Int. Cl.[4]: **C 07 D 471/04** // C07D213/79, C07D213/81 ,(C07D471/04, 221:00, 209:00)

(21) Anmeldenummer: **85810362.5**

(22) Anmeldetag: **05.08.85**

(54) **Verfahren zur Herstellung von Pyridin-2,3-dicarbonsäurederivaten und neue 1-Amino-1,2,3,4-tetrahydropyridin-2,3-dicarbonsäurederivate und 1,4-Dihydropyridin-2,3-dicarbonsäurederivate.**

(30) Priorität: **10.08.84 CH 3838/84**

(43) Veröffentlichungstag der Anmeldung: **19.02.86 Patentblatt 86/8**

(45) Bekanntmachung des Hinweises auf die Patenterteilung: **06.09.89 Patentblatt 89/36**

(84) Benannte Vertragsstaaten: **AT BE CH DE FR GB IT LI NL SE**

(56) Entgegenhaltungen: **EP-A- 0 041 623**

(73) Patentinhaber: **CIBA-GEIGY AG, Klybeckstrasse 141, CH-4002 Basel (CH)**

(72) Erfinder: **Waldner, Adrian, Dr., Holeeweg 39, CH-4123 Allschwil (CH)**

EP 0 172 140 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Pyridin-2,3-dicarbonsäurederivaten und neue 1-Amino-1,2,3,4-tetrahdropyridin-2,3-dicarbonsäurederivate und 1,4-Dihydropyridin-2,3-dicarbonsäurederivate, die bei der Durchführung des Verfahrens als Zwischenprodukte durchlaufen werden.

Aus der EP-A 0 041 623 sind 2-(2-Imidazolin-2-yl)-pyridin-3-carbonsäurederivate mit herbizider Wirkung bekannt, die ausgehend von entsprechenden Pyridin-2,3-dicarbonsäuren und Derivaten davon hergestellt werden können. Es steht jedoch bisher kein Verfahren zur Verfügung, das die Herstellung von Pyridin-2,3-dicarbonsäuren und Derivaten davon ausgehend von leicht zugänglichen Ausgangsmaterialien auf einfache Weise ermöglicht. Es ist das Ziel der vorliegenden Erfindung, diesem Mangel abzuhelfen und ein Verfahren bereitzustellen, nach dem Pyridin-2,3-dicarbonsäuren und ihre Derivate in einfacher Weise und in guten Ausbeuten aus billigen und leicht zugänglichen Ausgangsmaterialien hergestellt werden können.

Gegenstand der Erfindung ist ein Verfahren zur Herstellung von Pyridin-2,3-dicarbonsäurederivaten der Formel I

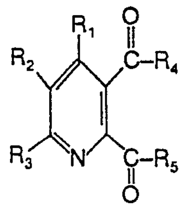

(I)

worin

$R_1$ Wasserstoff, $C_1$–$C_6$-Alkyl, $C_1$–$C_6$-Alkylthio oder $C_1$–$C_6$-Alkoxy, oder durch Hydroxy, Halogen, $C_1$–$C_4$-Alkoxy, Phenyl, Phenoxy, Cyano, Carboxyl oder $C_1$–$C_4$-Alkoxycarbonyl substituiertes $C_1$–$C_6$-Alkyl, $C_1$–$C_6$-Alkylthio oder $C_1$–$C_6$-Alkoxy, oder Phenyl oder Phenoxy, oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Phenoxy bedeutet,

$R_2$ die gleiche Bedeutung hat wie $R_1$ und zusätzlich Fluor, Chlor oder Brom bedeutet,

$R_3$ für Wasserstoff steht oder zusammen mit $R_2$ eine Trimethylen- oder Tetramethylengruppe bildet,

$R_4$ und $R_5$ unabhängig voneinander –OH, $-NH_2$, $-NHR_6$, $-NR_6R_7$ oder $-OR_8$ sind, wobei

$R_6$ und $R_7$ für $C_1$–$C_6$-Alkyl, $C_5$–$C_6$-Cycloalkyl, Allyl, Methallyl, Propargyl, Phenyl, Benzyl oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Benzyl stehen oder $R_6$ und $R_7$ zusammen $C_4$–$C_5$-Alkylen oder 3-Oxapentylen bedeuten,

$R_8$ für $C_1$–$C_6$-Alkyl, $C_5$–$C_6$-Cycloalkyl, Allyl, Methallyl, Propargyl, Phenyl oder Benzyl oder durch $C_1$–$C_4$-Alkyl, $C_1$–$C_4$-Alkoxy oder Halogen substituiertes Phenyl oder Benzyl steht, oder

$R_4$ und $R_5$ zusammen –O– oder $-NR_9-$ bedeuten, wobei

$R_9$ Wasserstoff, $C_1$–$C_6$-Alkyl, Allyl, Methallyl, Propargyl, Phenyl, Benzyl, $C_5$–$C_6$-Cycloalkyl; oder durch –OH, $-OR_{12}$, $-SR_{12}$, –COOH, $-COOR_8$, $-OCOR_{12}$, $-CONH_2$, $-CONHR_6$, $-CONR_6R_7$, Halogen oder Cyano substituiertes $C_1$–$C_{13}$-Alkyl; oder durch $C_1$–$C_4$-Alkyl, $C_1$–$C_4$-Alkoxy oder Halogen substituiertes Phenyl oder Benzyl und

$R_{12}$ $C_1$–$C_6$-Alkyl, Cyclohexyl oder Phenyl bedeutet,

dadurch gekennzeichnet, daß man ein 1-Amino-1,2,3,4-tetrahydropyridin-2,3-dicarbonsäurederivat der Formel II

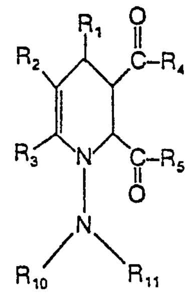

(II)

in welcher

$R_{10}$ und $R_{11}$ unabhängig voneinander $C_1$–$C_6$-Alkyl, $C_5$–$C_6$-Cycloalkyl, Benzyl oder Phenyl oder zusammen $C_4$–$C_5$-Alkylen oder 3-Oxapentylen bedeuten, durch Behandeln mit einer Säure und/oder durch thermische Behandlung bei mindestens 20 °C unter Abspaltung von $R_{10}R_{11}NH$ in ein 1,4-Dihydropyridin-2,3-dicarbonsäurederivat der Formel III

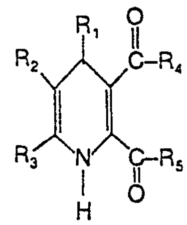

(III)

und anschließend dieses 1,4-Dihydropyridin-2,3-dicarbonsäurederivat der Formel III durch Oxidation in ein Pyridin-2,3-dicarbonsäurederivat umwandelt.

In obigen Formeln enthalten $R_1$ und $R_2$ als Alkyl, Alkylthio und Alkoxy 1 bis 6 C-Atome. Geeignete Substituenten für diese Reste sind Hydroxy, Halogen, besonders Fluor, Chlor und Brom, $C_1$–$C_4$-Alkoxy, Phenyl, Phenoxy, Cyano, Carboxyl und $C_1$–$C_4$-Alkoxycarbonyl. Bevorzugte Substituenten sind Hydroxy und Halogen. Beispiele für diese Reste sind: Methyl, Ethyl, n-Propyl, i-Propyl, n-Butyl, i-Butyl, t-Butyl, 1-Pentyl, 3-Pentyl, 1-Hexyl, 2-Hexyl, 3-Hexyl, Methoxy, Ethoxy, n-Propoxy, i-Propoxy, n-Butoxy, i-Butoxy, t-Butoxy, Methylthio, Ethylthio, Propylthio, Butylthio, Hydroxymethyl, 2-Hydroxyethyl, Fluormethyl, Trifluormethyl, 2-Cyanoethyl, 2-Chlorethyl, Brommethyl, Benzyl, Chlorbenzyl, Methoxymethyl, Ethoxymethyl, Methoxyethyl.

Geeignete Substituenten für $R_1$ und $R_2$ als Phenyl und Phenoxy sind z.B. Halogen, wie F, Cl und Br, CN, $C_1$–$C_4$-Alkyl, $C_1$–$C_4$-Alkoxy, $C_1$–$C_4$-Haloalkyl oder $C_1$–$C_4$-Cyanoalkyl. Beispiele sind Methylphenyl, Ethylphenyl, Methoxyphenyl, Ethoxyphenyl, Chlorphenyl, Fluorphenyl, Difluorphenyl, Bromphenyl, Chlorphenoxy, Methylphenoxy, Methoxyphenoxy, Fluormethylphenyl, Difluormethylphenyl, Trifluormethylphenyl, Chlormethylphenyl, Cyanomethylphenyl, 2-Cyanoethylphenyl, Trifluormethylphenoxy und Cyanomethylphenoxy.

$R_4$ und $R_5$ sind bevorzugt –OH oder $OR_8$ und zusammen –O– oder $–NR_9–$. $R_6$ und $R_7$ enthalten als Alkyl 1 bis 6, besonders 1 bis 4 C-Atome. Als Cycloalkyl sind $R_6$ und $R_7$ Cyclopentyl oder Cyclohexyl. $R_6$ und $R_7$ zusammen sind als Alkylen, Pentamethylen oder Tetramethylen und als Oxaalkylen 3-Oxapentylen. $R_6$ und $R_7$ sind als Aryl bzw. Aralkyl Phenyl oder Benzyl, die wie $R_1$ und $R_2$ als Phenyl substituiert sein können.

$R_8$ kann als Alkyl linear oder verzweigt sein und 1 bis 6 C-Atome enthalten. $R_8$ ist als Cycloalkyl Cyclopentyl oder Cyclohexyl. $R_8$ als Aryl bzw. Aralkyl ist Phenyl oder Benzyl das substituiert sein kann, z.B. durch $C_1$–$C_4$-Alkyl, $C_1$–$C_4$-Alkoxy oder Halogen wie Fluor, Chlor oder Brom.

Geeignete Substituenten für $R_9$ sind beispielsweise –OH, $–OR_{12}$, $–SR_{12}$, worin $R_{12}$ $C_1$–$C_6$-Alkyl, Cyclohexyl oder Phenyl ist, –COOH, $–COOR_8$, $–OCOR_{12}$, $–CONH_2$, $–CONHR_6$, $–CONR_6R_7$ und Halogen, wie z.B. Fluor, Chlor oder Brom und Cyano. $R_9$ enthält als Alkyl 1 bis 6 C-Atome. Beispiele sind zuvor erwähnt worden.

$R_9$ als Cycloalkyl ist Cyclohexyl oder Cyclopentyl. Beispiele für $R_9$ als substituierte Reste sind Hydroxymethyl, 2-Hydroxyethyl, Methoxymethyl, Methoxyethyl, Ethoxyethyl, Phenoxyethyl, Dimethylamino, Diethylamino, Methoxycarbonylmethyl, Ethoxycarbonylethyl, Propoxycarbonylmethyl, Phenoxycarbonylmethyl, Benzoyloxymethyl, Acetyloxyethyl, Methylaminocarbonylmethyl, Dimethylaminocarbonylethyl, Chlormethyl, 2-Chlorethyl, Cyanomethyl, 2-Cyanoethyl, 2-Cyanopropyl, 2-[2-Cyano-3-methyl]butyl und 2-[2-Carbamoyl-3-methyl]butyl.

$R_4$ und $R_5$ sind vorzugsweise –OH, $–NH_2$, $–NHR_6$, $–NR_6R_7$ oder $–OR_8$, worin $R_6$ und $R_7$ $C_1$–$C_6$-Alkyl, Cyclopentyl, Cyclohexyl, Phenyl oder Benzyl oder $R_6$ und $R_7$ zusammen Tetramethylen, Pentamethylen oder 3-Oxapentylen bedeuten, oder $R_4$ und $R_5$ sind zusammen –O– oder $–NR_9–$, worin $R_9$ $C_1$–$C_6$-Alkyl, $C_1$–$C_{13}$-Cycloalkyl, $C_1$–$C_{13}$-Aminocarbonylalkyl oder Phenyl ist.

$R_{10}$ und $R_{11}$ als Alkyl können linear oder verzweigt sein und enthalten 1 bis 6, besonders 1 bis 4 C-Atome. Besonders bevorzugte Reste $R_{10}$ und $R_{11}$ sind Ethyl oder Methyl. Als Cycloalkyl sind $R_{10}$ und $R_{11}$ Cyclopentyl oder Cyclohexyl, als Aryl Phenyl und als Aralkyl Benzyl. In einer bevorzugten Ausführungsform sind $R_{10}$ Phenyl und $R_{11}$ $C_1$–$C_6$-Alkyl. $R_{10}$ und $R_{11}$ zusammen sind als Alkylen Tetramethylen oder Pentamethylen und als Oxaalkylen 3-Oxapentylen.

In einer bevorzugten Ausführungsform stehen $R_4$ und $R_5$ für einen Rest der Formel

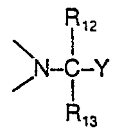,

worin Y für –CN oder $–CONH_2$ steht und $R_{12}$ und $R_{13}$ unabhängig voneinander für ein Wasserstoffatom oder lineares oder verzweigtes $C_1$–$C_6$-Alkyl stehen. Bevorzugt sind $R_{12}$ und $R_{13}$ lineares oder verzweigtes $C_1$–$C_4$-Alkyl.

In einer besonders bevorzugten Untergruppe stehen $R_4$ und $R_5$ für

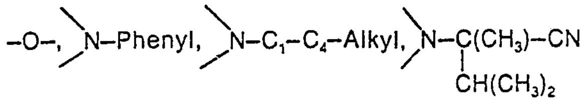

oder 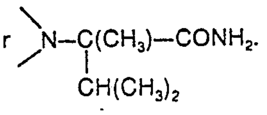.

Das erfindungsgemäße Verfahren wird vorteilhaft in einem inerten Lösungsmittel durchgeführt.

Geeignete Lösungsmittel sind solche, die gegenüber den Reaktionsteilnehmern inert sind. Besonders geeignet sind polare, protische und aprotische Lösungsmittel, die einzeln oder in Mischungen aus mindestens zwei Lösungsmitteln verwendet werden können. Beispiele sind: Ether wie Dibutylether, Tetrahydrofuran, Dioxan, Ethylenglykol, Dimethylethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton und ε-Valerolacton, Carbonsäureamide und Lactame wie Formamid, Acetamid, N-Methylformamid, N,N-Dimethylformamid, N,N-Diethylformamid, N,N-Dimethylacetamid, N,N-Diethylacetamid, γ-Butyrolactam, N-Methylpyrrolidon, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylensulfon, tert.Amine, wie Trimethylamin, Triethylamin, N-Methylpyrrolidin, N-Methylpiperidin, N-Methylmorpholin, substituierte Benzole, wie Toluol, Chlorbenzol, Nitrobenzol, Nitrile, wie Acetonitril, Alkohole, wie Methanol, Ethanol oder Propanol.

Die 1-Amino-1,2,3,4-Tetrahydropyridin-2,3-dicarbonsäurederivate der Formel II sind teilweise bekannt und können nach an sich bekannten Verfahren durch Umsetzung von α,β-ungesättigten Hydrazonen der Formel IV

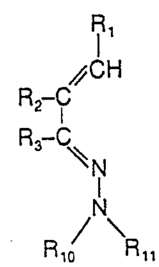 (IV),

in welcher $R_1$, $R_2$, $R_3$, $R_{10}$ und $R_{11}$ die oben angegebene Bedeutung haben, wie Ethen-1,2-dicarbonsäurederivaten der Formel V

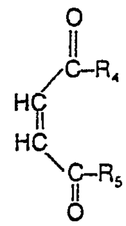
(V),

in welcher $R_4$ und $R_5$ die oben angegebene Bedeutung haben, hergestellt werden (vgl. Tetrahedron Letters, Vol. 23, No. 32, pp. 3261–3264 (1982)).

Die neuen 1-Amino-1,2,3,4-tetrahydropyridin-2,3-dicarbonsäurederivate sind ebenfalls Gegenstand der Erfindung. Sie entsprechen der Formel II gemäß obiger Definition mit Ausnahme derjenigen Verbindungen, in welchen $R_4$ und $R_5$ OH, $OCH_3$, $OC_2H_5$ oder zusammen –O– bedeuten, wenn gleichzeitig $R_1$ für Wasserstoff und $R_2$ für Methyl steht. Bevorzugte Verbindungen der Formel II sind solche, worin $R_4$ und $R_5$ zusammen $-NR_9-$ bedeuten. Sie entsprechen der Formel IIa

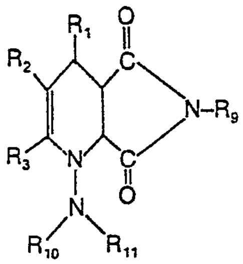
(IIa)

in welcher $R_1$, $R_2$, $R_3$, $R_9$, $R_{10}$ und $R_{11}$ die oben angegebene Bedeutung haben.

Die 1,4-Dihydropyridin-2,3-dicarbonsäurederivate der Formel III sind ebenfalls neu und bilden einen weiteren Gegenstand der vorliegenden Erfindung. Bevorzugte Verbindungen der Formel III sind solche, worin $R_4$ und $R_5$ zusammen für –O– oder $-NR_9-$ stehen. Diese Verbindungen entsprechen den Formeln IIIa und IIIb

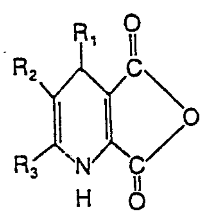
(IIIa)
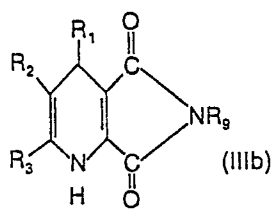
(IIIb)

worin $R_1$, $R_2$, $R_3$ und $R_9$ die oben angegebene Bedeutung haben.

Die Abspaltung von $HNR_{10}R_{11}$ aus
1-Amino-1,2,3,4-tetrahydropyridin-2,3-dicarbon
säurederivaten
der Formel II kann in einem weiten Temperaturbereich durchgeführt werden. Geeignete Temperaturen liegen im Bereich von 20–200 °C. Vorzugsweise wird die Reaktion von einer Temperatur von 40–150 °C in einem inerten Lösungsmittel durchgeführt. Bevorzugte Lösungsmittel sind Toluol und Äthanol. Geeignete Säuren in deren Gegenwart die Aminabspaltung durchgeführt werden kann, sind insbesondere organische Säuren, wie Ameisensäure, Essigsäure, Propionsäure oder Oxalsäure. Besonders geeignet sind saure Silikate, wie z.B. Kieselgel. Besonders vorteilhaft wird die Reaktion durch Erhitzen eines
1-Amino-1,2,3,4-tetrahydropyridin-2,3-dicarbonsäurederivate
wie etwa der doppelten Menge Kieselgel in Toluol bei Rückflußtemperatur durchgeführt.

Nach einer Variante des erfindungsgemäßen Verfahrens können die durch Abspaltung von $HNR_{10}R_{11}$ aus den 1-Amino-1,2,3,4-tetrahydropyridin-2,3-dicarbonsäurederivaten der Formel II erhältlichen 1,4-Dihydropyridin-2,3-dicarbonsäurederivate der Formel III auch durch Umsetzung von α,β-ungesättigten Hydrazonen der Formel IV mit Ethylen-1,2-dicarbonsäurederivaten der Formel V ohne Isolierung der intermediär gebildeten
1-Amino-1,2,3,4-tetrahydropyridin-2,3-dicarbonsäurederivate
der Formel II direkt hergestellt werden. Diese Variante wird in der Weise durchgeführt, daß man ein α,β-ungesättigtes Hydrazon der Formel IV und ein Ethen-1,2-dicarbonsäurederivat der Formel V in einem inerten Lösungsmittel auf Temperaturen von 40–150 °C erhitzt und das gebildete 1,4-Dihydropyridin-2,3-dicarbonsäurederivat der Formel III anschließend durch Oxidation in ein Pyridin-2,3-dicarbonsäurederivat der Formel I überführt. Besonders geeignete Lösungsmittel sind Acetonitril oder niedere Alkanole, wie Methanol, Ethanol oder Propanol. Die Reaktionszeit beträgt je nach Temperatur in der Regel 10–25 Stunden, insbesondere 15–20 Stunden. Diese Reaktionszeiten können jedoch durch Zugabe der vorgenannten Säuren, insbesondere durch Zugabe eines sauren Silikats, wesentlich unterschritten werden.

Geeignete Oxidationsmittel zur Überführung eines 1,4-Dihydropyridin-2,3-dicarbonsäurederivats der Formel III in ein Pyridin-2,3-dicarbonsäurederivat der Formel I sind beispielsweise, Sauerstoff, Wasserstoffperoxid, Mangandioxid (Braunstein), Chromsäure, Lösungen vom Iod in einem organischen Lösungsmittel und insbesondere Luft. Chromsäure kann zweckmäßig als Natrium- oder Kaliumdichromat gegebenenfalls zusammen mit organischen Säuren eingesetzt werden. Die Oxidation mit Luft kann vorteilhaft in Gegenwart von Katalysatoren, wie Palladium oder Platin, erfolgen. Die Reaktion wird zweckmäßig in einem inerten Lösungsmittel in Gegenwart von organischen Säuren, insbesondere Essigsäure, durchgeführt, wobei die organischen Säuren direkt als Lösungsmittel dienen können. Die Reaktionstemperatur beträgt im allgemeinen 20–200 °C, vorzugsweise 30–100 °C.

Die Oxidation von 1,4-Dihydropyridin-2,3-carbonsäurederivaten der Formel III zu Pyridin-2,3-dicarbonsäurederivaten der Formel I kann in besonders vorteilhafter Weise durch Erwärmen mit der im wesentlichen äquimolaren Menge Mangandioxid in Essigsäure durchgeführt werden. Dabei betragen die Reaktionstemperaturen in der

Regel 50–100 °C und die Reaktionszeiten $^{1}/_{2}$ bis 1 Stunde.

Die Verbindungen der Formel I können nach dem erfindungsgemäßen Verfahren durch Verwendung entsprechender Ausgangsmaterialien direkt hergestellt werden. Es ist jedoch auch möglich, direkt erhaltene Verbindungen der Formel I durch Derivatisierung innerhalb des durch die Formel I definierten Umfanges in andere Verbindungen der Formel I überzuführen. So können beispielsweise aus Anhydriden der Formel I die freien Säuren und deren Halbester, Ester, Halbamide und Amide hergestellt werden.

Mit dem erfindungsgemäßen Verfahren gelangt man unter Verwendung einfach zugänglicher Ausgangsprodukte auf einem kurzen Syntheseweg unter milden Reaktionsbedingungen in hohen Ausbeuten zu reinen Pyridin-2,3-dicarbonsäurederivaten der Formel I das Verfahren ist insbesondere für die Durchführung im technischen Maßstab geeignet.

Die Verbindungen der Formel I sind wertvolle Zwischenprodukte, besonders zur Herstellung von Herbiziden, wie sie beispielsweise in der EP-A 0 041 623, EP-A 0 041 624 und EP-A 0 095 104 beschrieben sind.

Die nachfolgenden Beispiele erläutern die Erfindung näher:

Beispiel a

Herstellung von

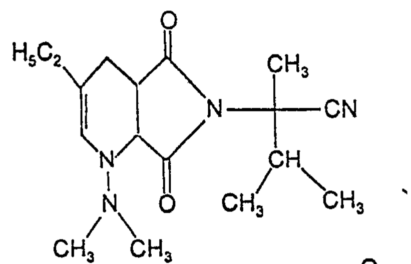

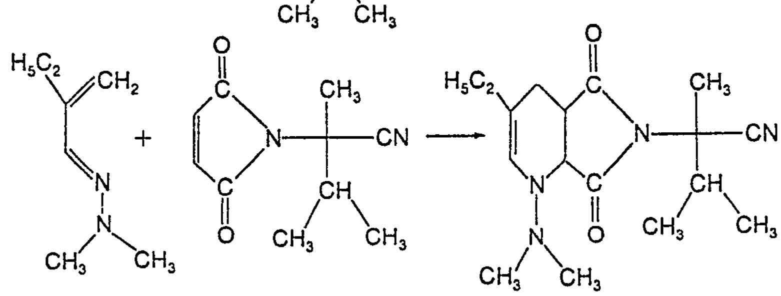

16,5 g Hydrazon und 10,8 g Imid werden in 100 ml Acetonitril 3,5 h auf 60 °C erwärmt. Die gelbe Lösung liefert nach Eindampfen quantitativ ein oranges Öl.

NMR($CDCl_3$): 6,0 (H–C=C); 2,55 ($N(CH_3)_2$); 3,95 (N–CH–C=O)

Beispiel b

Durch 1–3 stündiges Erwärmen von äquimolaren Mengen Hydrazon und Imid in der 2–5 fachen Menge Ethanol auf 70 °C und Abdampfen des Lösungsmittels werden folgende Verbindungen erhalten:

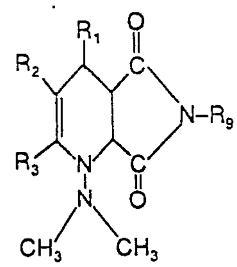

| $R_1$ | $R_2$ | $R_9$ | |
|---|---|---|---|
| $-[-CH_2-]-$ | | $C_6H_5$ | |
| H | $n-C_3H_7$ | $C_6H_5$ | |
| $CH_3$ | $n-C_3H_7$ | $C_6H_5$ | |
| $p-Cl-C_6H_4$ | $C_2H_5$ | $C_6H_5$ | |
| $CH_3$ | $CH_3COOCH_2$ | $C_6H_5$ | |
| H | $C_2H_5$ | $C_6H_5$ | Smp. 113–15° C |
| H | H | 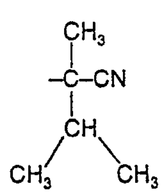 | |

| $R_1$ | $R_2$ | $R_9$ | |
|---|---|---|---|
| $-[-CH_2-]-$ | | 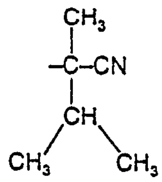 | |
| n-$C_3H_7$ | $C_2H_5$ | 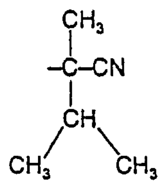 | |
| $C_2H_5$ | $CH_3$ | 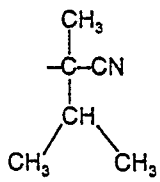 | |
| $CH_3$ | $CH_3COOCH_2$ | 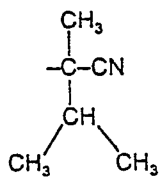 | |
| H | n-$C_4H_9$ | 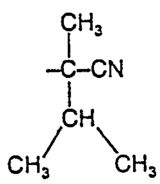 | Öl |
| H | i-$C_3H_7$ | 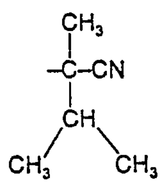 | Öl |
| 4-Cl-$C_6H_4$ | $CH_3$ | 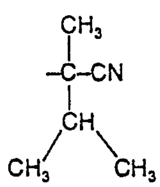 | Öl |
| H | $CH_3$ | 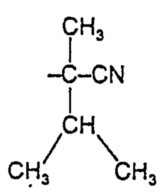 | Öl |
| $CH_3$ | F | 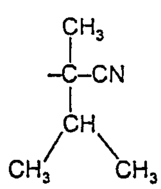 | Öl |

| $R_1$ | $R_2$ | $R_9$ |
|---|---|---|
| H | F | 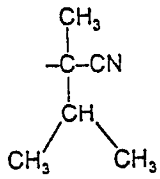 |

Beispiel 1:

a) Herstellung von

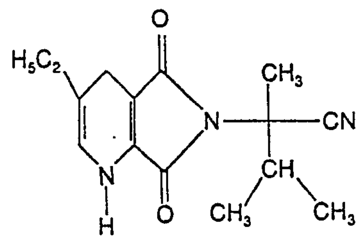

27,3 g Tetrahydropyridin gemäß Beispiel a) werden in 300 ml Toluol gelöst, mit 30 g Kieselgel versetzt und auf Rückflußtemperatur erhitzt. Nach $1^1/_2$ h wird eingedampft und über wenig Kieselgel mit Toluol/Essigester (4:1) eluiert. Das Eluat wird eingedampft und es verbleiben 19 g rote Kristalle, welche sich langsam zersetzen.

NMR ($CDCl_3$): 5,90 (H=C); 6,70 (NH, mit $D_2O$ austauschbar).

b) Herstellung von

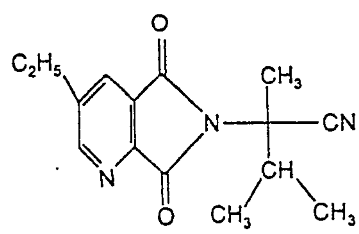

6 g Dihydropyridin werden in 40 ml Essigsäure gelöst und auf 45 °C erwärmt. Man leitet eine halbe Stunde lang Luft durch die Lösung, wobei eine gelbe Lösung entsteht. Man dampft ein und verteilt zwischen Chloroform und 2 n Soda. Nach Trocknen der organischen Phase wird eingedampft. Der Rückstand wird aus Ether umkristallisiert. Man erhält 3,1 g Produkt mit einem Smp. 73–73,5 °C.

Elementaranalyse:

| | | | |
|---|---|---|---|
| Ber. | C 66,40 | H 6,32 | N 15,49 |
| Gef. | C 66,30 | H 6,35 | N 15,46. |

Beispiel 2:

Analog Beispiel 1a werden folgende 1,4-Dihydropyridin-2,3-dicarbonsäurederivate hergestellt:

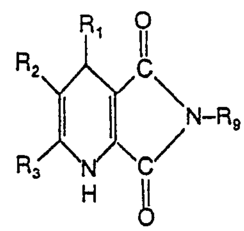

| $R_1$ | $R_2$ | $R_9$ | Smp. (°C) |
|---|---|---|---|
| $n-C_3H_7$ | $CH_3$ | $C_6H_5$ | 144–150 |
| H | $n-C_3H_7$ | $C_6H_5$ | 174–176 |
| $-[-CH_2-]_4-$ | | $C_6H_5$ | 170–173 |
| $C_6H_5$ | $C_2H_5$ | $C_6H_5$ | 171–173 |
| $CH_3$ | $CH_3COOCH_2$ | $C_6H_5$ | 125–128 |
| $3-NO_2C_6H_4$ | $CH_3$ | $C_6H_5$ | 199–202 |
| H | $C_2H_5$ | $C_6H_5$ | |
| $2,3-Cl-C_6H_3$ | $C_2H_5$ | $C_6H_5$ | |

| $R_1$ | $R_2$ | $R_9$ | Smp. (°C) |
|---|---|---|---|
| H | $n\text{-}C_3H_7$ | 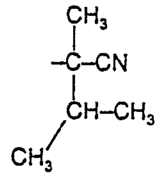 | Öl |
| $C_6H_5$ | $C_6H_5$ | 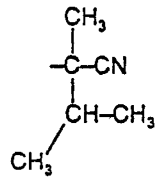 | 135–136 |
| $C_2H_5$ | $CH_3$ | 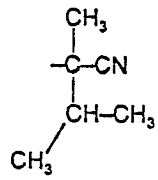 | Öl |
| $n\text{-}C_3H_7$ | $C_2H_5$ | 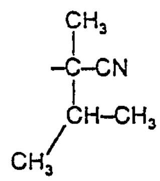 | Öl |
| $-[-CH_2-]_4-$ | | 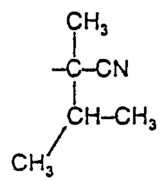 | Öl |
| $C_6H_5$ | $CH_3$ | 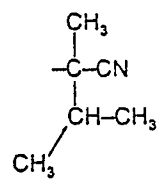 | 168–169 |
| $CH_3$ | $CH_3COOCH_2$ | 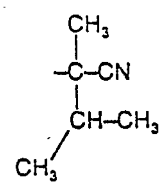 | Öl |
| H | $n\text{-}C_4H_9$ | 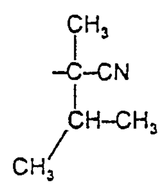 | Öl |
| H | $n\text{-}C_3H_7$ | 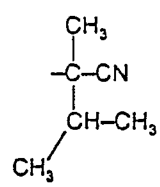 | Öl |

| $R_1$ | $R_2$ | $R_9$ | Smp. (°C) |
|---|---|---|---|
| $4-Cl-C_6H_4$ | $CH_3$ | 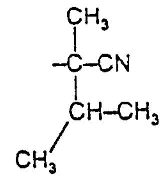 | Öl |
| $3-NO_2-C_6H_4$ | $CH_3$ | 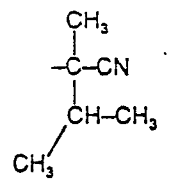 | 161–162 |
| $C_2H_5$ | $CH_3$ | 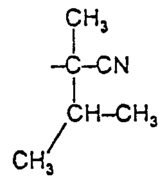 | Öl |
| $CH_3$ | F | 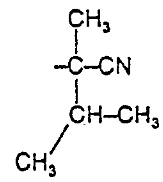 | 145–147 |
| H | $n-C_3H_7$ | 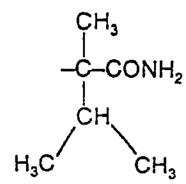 | Harz |
| H | F | 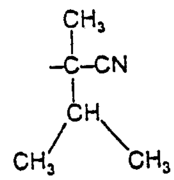 | Öl |
| H | $CH_3$ | 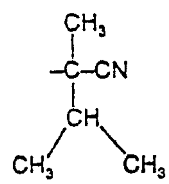 | |

Beispiel 3:

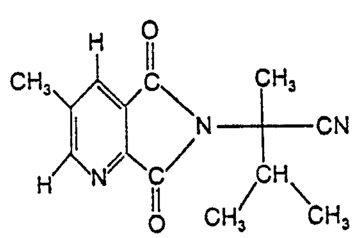

140,7 g Dihydropyridin (Vgl. Bsp. 2, letzte Zeile) werden in 800 ml Essigsäure suspendiert und auf 60 °C erwärmt. Dann werden 71 g Mangandioxid portionenweise eingetragen und anschließend 1/2 Stunde bei 60 °C nachgerührt. Nach Abkühlen der Mischung auf Raumtemperatur wird filtriert und eingedampft. Der Rückstand wird zwischen Chloroform und Sodalösung verteilt. Die organische Phase wird abgetrennt und nach Trocknen über Magnesiumsulfat eingedampft. Der Rückstand

wird mit Ether digeriert. Man erhält 119 g der obigen Verbindung als beiges Pulver vom Smp. 119–20°.

Auf analoge Weise werden folgende Verbindungen hergestellt:

Herstellung von

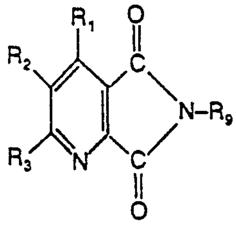

| $R_1$ | $R_2$ | $R_3$ | $R_9$ | Smp. (° C) |
|---|---|---|---|---|
| $n\text{-}C_3H_7$ | $CH_3$ | H | $C_6H_5$ | 177–179 |
| H | $n\text{-}C_3H_7$ | H | $C_6H_5$ | 153–154 |
| $-[-CH_2-]_4-$ | | H | $C_6H_5$ | 149–150 |
| $C_6H_5$ | $C_2H_5$ | H | $C_6H_5$ | |
| $CH_3$ | $CH_3COOCH_2$ | H | $C_6H_5$ | 118–120 |
| H | $C_6H_5$ | H | $C_6H_5$ | 190–191 |
| $CH_3$ | $CH_3$ | H | $C_6H_5$ | 244–246 |
| $-[-CH_2-]_4-$ | | H | $C_6H_5$ | 149–150 |
| H | $n\text{-}C_3H_7$ | H | 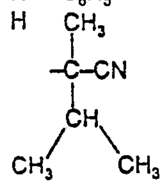 | 59–60 |
| $C_6H_5$ | $CH_3$ | H | 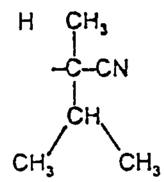 | 105–107 |
| $n\text{-}C_3H_7$ | $C_2H_5$ | H | 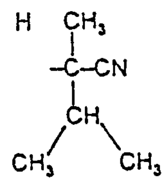 | 76–77 |
| $-[-CH_2-]_4-$ | | H | 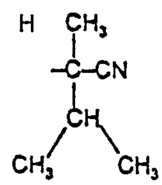 | 121–125 |
| $C_6H_5$ | $CH_3$ | H | 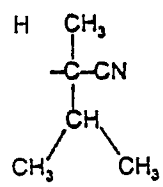 | 169–170 |
| $C_6H_5$ | $CH_3$ | | 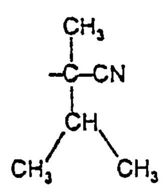 | 142–143 |

| $R_1$ | $R_2$ | $R_3$ | $R_9$ | Smp. (° C) |
|---|---|---|---|---|
| $CH_3$ | $CH_3COOCH_2$ | H | 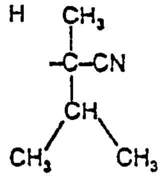 | Öl |
| H | n-$C_4H_9$ | H | 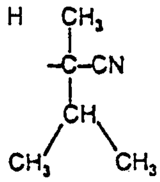 | Öl |
| H | i-$C_3H_7$ | H | 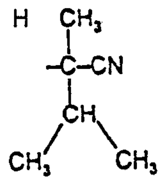 | 95–96 |
| 4-Cl–$C_6H_4$ | $CH_3$ | H | 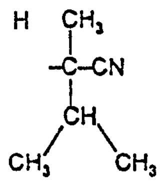 | 112–114 |
| 3-$NO_2$–$C_6H_4$ | $CH_3$ | H | 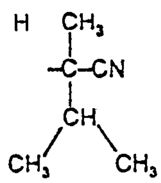 | 164–166 |
| H | $CH_3$ | H | 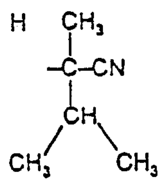 | 119–120 |
| $CH_3$ | F | H | 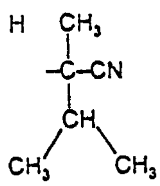 | Öl |
| H | $C_2H_5$ | H | 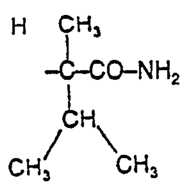 | 90–94 |
| H | F | | 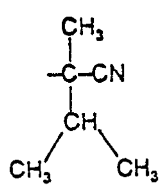 | |

Beispiel 4:

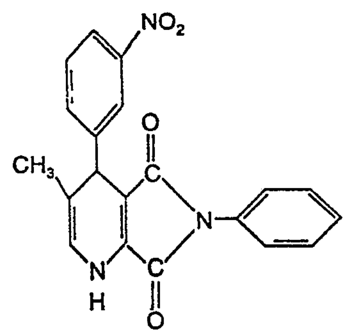

30,3 g des N,N-Dimethylhydrazons von 3-(3-Nitrophenyl)-2-methyl-2-propen-1-al (3-(3-Nitrophenyl)-methacrolein) und 22,5 g N-Phenylmaleinimid werden in 260 ml Acetonitril 16 Stunden auf Rückflußtemperatur erhitzt. Danach wird mit Aktivkohle behandelt und filtriert. Nach dem Abdampfen des Lösungsmittels wird der Rückstand mit Äther/Hexan digeriert und abgedampft. Man erhält 20,5 g der obigen Verbindung, vom Smp. 199–202 °C.

Auf analoge Weise erhält man aus dem N,N-Dimethylhydrazon des 3-(3-Nitrophenyl)-methacroleins und N-(1-cyano-1,3-dimethylpropyl)-maleinimid die Verbindung der Formel

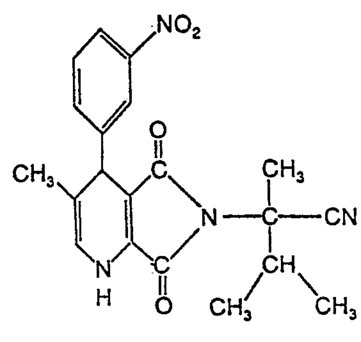

vom Smp. 161–162 °C.

**Patentansprüche für die Vertragsstaaten: DE, GB, FR, CH, LI, IT, NL, BE, SE**

1. Verfahren zur Herstellung von Pyridin-2,3-dicarbonsäurederivaten der Formel I

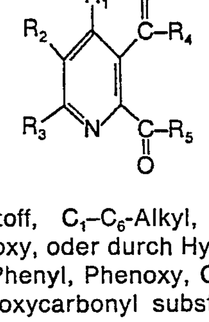

(I)

worin

$R_1$ Wasserstoff, $C_1$–$C_6$-Alkyl, $C_1$–$C_6$-Alkylthio oder $C_1$–$C_6$-Alkoxy, oder durch Hydroxy, Halogen, $C_1$–$C_4$-Alkoxy, Phenyl, Phenoxy, Cyano, Carboxyl oder $C_1$–$C_4$-Alkoxycarbonyl substituiertes $C_1$–$C_4$-Alkyl, $C_1$–$C_6$-Alkylthio oder $C_1$–$C_6$-Alkoxy, oder Phenyl oder Phenoxy, oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Phenoxy bedeutet,

$R_2$ die gleiche Bedeutung hat wie $R_1$ und zusätzlich Fluor, Chlor oder Brom bedeutet,

$R_3$ für Wasserstoff steht oder zusammen mit $R_2$ eine Trimethylen- oder Tetramethylengruppe bildet,

$R_4$ und $R_5$ unabhängig voneinander –OH, –$NH_2$, –$NHR_6$, –$NR_6R_7$ oder –$OR_8$ sind, wobei

$R_6$ und $R_7$ für $C_1$–$C_6$-Alkyl, $C_5$–$C_6$-Cycloalkyl, Allyl, Methallyl, Propargyl, Phenyl, Benzyl oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Benzyl stehen oder $R_6$ und $R_7$ zusammen $C_4$–$C_5$-Alkylen oder 3-Oxapentylen bedeuten,

$R_8$ für $C_1$–$C_6$-Alkyl, $C_5$–$C_6$-Cycloalkyl, Allyl, Methallyl, Propargyl, Phenyl oder Benzyl oder durch $C_1$–$C_4$-Alkyl, $C_1$–$C_4$-Alkoxy oder Halogen substituiertes Phenyl oder Benzyl steht, oder

$R_4$ und $R_5$ zusammen –O– oder –$NR_9$– bedeuten, wobei

$R_9$ Wasserstoff, $C_1$–$C_6$-Alkyl, Allyl, Methallyl, Propargyl, Phenyl, Benzyl, $C_5$–$C_6$-Cycloalkyl; oder durch –OH, –$OR_{12}$, –$SR_{12}$, –COOH, –$COOR_8$, –$OCOR_{12}$, –$CONH_2$, –$CONHR_6$, –$CONR_6R_7$, Halogen oder Cyano substituiertes $C_1$–$C_{13}$-Alkyl; oder durch $C_1$–$C_4$-Alkyl, $C_1$–$C_4$-Alkoxy oder Halogen substituiertes Phenyl oder Benzyl und

$R_{12}$ $C_1$–$C_6$-Alkyl, Cyclohexyl oder Phenyl bedeutet,

dadurch gekennzeichnet, daß man ein 1-Amino-1,2,3,4-tetrahydropyridin-2,3-dicarbonsäurederivat der Formel II

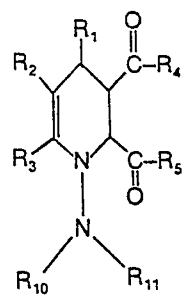

(II)

in welcher

$R_{10}$ und $R_{11}$ unabhängig voneinander $C_1$–$C_6$-Alkyl, $C_5$–$C_6$-Cycloalkyl, Benzyl oder Phenyl oder zusammen $C_4$–$C_5$-Alkylen oder 3-Oxapentylen bedeuten, durch Behandeln mit einer Säure und/oder durch thermische Behandlung bei mindestens 20 °C unter Abspaltung von $R_{10}R_{11}NH$ in ein 1,4-Dihydropyridin-2,3-dicarbonsäurederivat der Formel III

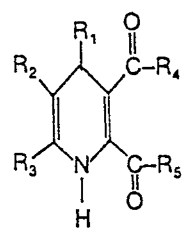

(III)

und anschließend dieses 1,4-Dihydropyridin-2,3-dicarbonsäurederivat der Formel III durch Oxidation in ein Pyridin-2,3-dicarbonsäurederivat umwandelt.

2. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III bei Temperaturen von 20–200 °C durchführt.

3. Verfahren nach Anspruch 2, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III bei 40–150 °C in einem inerten Lösungsmittel durchführt.

4. Verfahren nach Anspruch 3, dadurch gekennzeichnet, daß man als inerte Lösungsmittel Toluol oder Ethanol verwendet.

5. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III in Gegenwart einer organischen Säure aus der Gruppe Ameisensäure, Essigsäure oder Propionsäure durchführt.

6. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III in Gegenwart eines sauren Silikats durchführt.

7. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III durch Erhitzen des 1-Amino-1,2,3,4-tetrahydropyridin-2,3-dicarbonsäurederivates der Formel II mit etwa der doppelten Menge Kieselgel in Toluol bei Rückflußtemperatur durchführt.

8. Verfahren zur Herstellung von Pyridin-2,3-dicarbonsäurederivaten der Formel I gemäß Anspruch 1, dadurch gekennzeichnet, daß man ein α,β-ungesättigtes Hydrazon der Formel IV

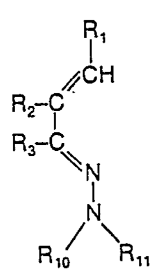 (IV),

in welcher $R_1$, $R_2$, $R_3$, $R_{10}$ und $R_{11}$ die unter Formeln I und II angegebene Bedeutung haben, und ein Ethen-1,2-dicarbonsäurederivat der Formel V

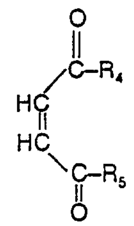 (V),

in welcher $R_4$ und $R_5$ die unter Formel I angegebene Bedeutung haben, in einem inerten Lösungsmittel auf Temperaturen von 40–150 °C erhitzt und das gebildete 1,4-Dihydropyridin-2,3-dicarbonsäurederivat der Formel III anschließend durch Oxidation in ein Pyridin-2,3-dicarbonsäurederivat der Formel I überführt.

9. Verfahren nach Anspruch 8, dadurch gekennzeichnet, daß man als Lösungsmittel Acetonitril oder ein niederes Alkanol verwendet.

10. Verfahren nach Ansprüchen 1 und 8, dadurch gekennzeichnet, daß man zur Überführung des 1,4-Dihydropyridin-2,3-dicarbonsäurederivates der Formel III in ein Pyridin-2,3-dicarbonsäurederivat der Formel I Luft, Sauerstoff, Wasserstoffperoxid, Mangandioxid, Chromsäure, als Oxidationsmittel verwendet.

11. Verfahren nach Anspruch 10, dadurch gekennzeichnet, daß man die Oxidation des 1,4-Dihydropyridin-2,3-dicarbonsäurederivates der Formel II zum Pyridin-2,3-dicarbonsäurederivat der Formel I mit Luft durchführt.

12. Verfahren nach Anspruch 10, dadurch gekennzeichnet, daß man die Oxidation des 1,4-Dihydropyridin-2,3-dicarbonsäurederivates der Formel III zum Pyridin-2,3-dicarbonsäurederivat der Formel I in Gegenwart einer organischen Säure bei 20–200 °C durchführt.

13. Verfahren nach Anspruch 10, dadurch gekennzeichnet, daß man die Oxidation des 1,4-Dihydropyridin-2,3-dicarbonsäurederivates der Formel III zum Pyridin-2,3-dicarbonsäurederivat der Formel I durch Erwärmen mit der im wesentlichen äquimolaren Menge Mangandioxid in Essigsäure bei Temperaturen von 50–100 °C durchführt.

14. Verfahren nach Ansprüchen 1 und 8, dadurch gekennzeichnet, daß man ein 1-Amino-1,2,3,4-tetrahydropyridin-2,3-dicarbonsäurederivat der Formel II bzw. ein Ethen-1,2-dicarbonsäurederivat der Formel V verwendet, in welcher $R_4$ und $R_5$ für einen Rest der Formel verwendet, in welcher $R_4$ und $R_5$ für einen Rest der Formel

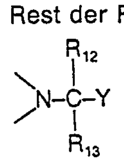 ,

stehen, worin Y für $-CN$ oder $-CONH_2$ steht und $R_{12}$ und $R_{13}$ unabhängig voneinander für ein Wasserstoffatom oder lineares oder verzweigtes $C_1$–$C_6$-Alkyl stehen.

15. Verfahren nach Ansprüchen 1 und 8, dadurch gekennzeichnet, daß man ein 1-Amino-1,2,3,4-tetrahydropyridin-2,3-dicarbonsäurederivat

der Formel II bzw. ein Ethen-1,2-dicarbonsäurederivat der Formel V verwendet, worin $R_4$ und $R_5$ zusammen für

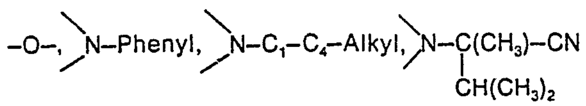

oder 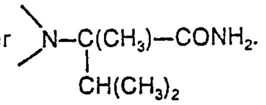

stehen.

16. Verfahren nach Ansprüchen 1 und 8, dadurch gekennzeichnet, daß man Pyridin-2,3-dicarbonsäurederivate der Formel I herstellt, in welchen $R_1$ Wasserstoff oder Methyl, $R_2$ Wasserstoff, $C_1$–$C_6$-Alkyl, Fluor, Chlor, Brom, $C_1$–$C_6$-Alkoxy, $C_1$–$C_4$-Alkylthio, Phenoxy, $C_1$–$C_6$-Hydroxyalkyl, $C_1$–$C_6$-Halogenalkyl, unsubstituiertes oder durch $C_1$–$C_4$-Alkyl, $C_1$–$C_4$-Alkoxy oder Halogen substituiertes Phenyl, $R_3$ Wasserstoff und $R_4$ und $R_5$ einzeln je –OH oder –$OR_8$ oder zusammen –O– oder –$NR_9$– bedeuten.

17. Verfahren nach Ansprüchen 1 und 8, dadurch gekennzeichnet, daß man Pyridin-2,3-dicarbonsäurederivate der Formel I herstellt in welchen $R_1$ Wasserstoff, $R_2$ $C_1$–$C_6$-Alkyl, $R_3$ Wasserstoff und $R_4$ und $R_5$ einzeln je –OH oder –$OR_8$ oder zusammen eine Gruppe

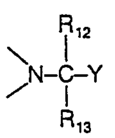

bedeuten, wobei $R_8$, $R_{12}$ und $R_{13}$ jeweils die unter Formeln I und II angegebene Bedeutung haben und Y eine Cyano- oder eine Carbamoylgruppe bedeutet.

18. 1-Amino-1,2,3,4-tetrahydropyridin-2,3-dicarbonsäurederivate der Formel II

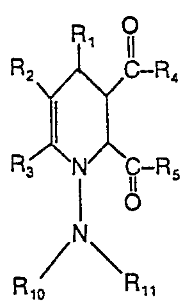

(II)

worin $R_1$, $R_2$, $R_3$, $R_4$, $R_5$, $R_{10}$ und $R_{11}$ die in Anspruch 1 angegebene Bedeutung haben, mit der Maßgabe, daß $R_4$ und $R_5$ nicht OH, $OCH_3$, $OGH_5$ oder zusammen –O– bedeuten, wenn $R_1$ für Wasserstoff und $R_2$ für Methyl steht.

19. 1-Amino-1,2,3,4-tetrahydroxypyridin-2,3-dicarbonsäurederivate gemäß Anspruch 18 der Formel IIa

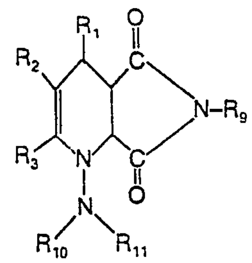

(IIa)

worin $R_1$, $R_2$, $R_3$, $R_9$, $R_{10}$ und $R_{11}$ die in Anspruch 1 angegebene Bedeutung haben.

20. 1,4-Dihydropyridin-2,3-dicarbonsäurederivate der Formel III

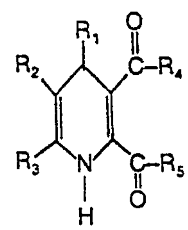

(III)

worin $R_1$, $R_2$, $R_3$, $R_4$ und $R_5$ die in Anspruch 1 angegebene Bedeutung haben.

21. 1,4-Dihydropyridin-2,3-dicarbonsäurederivate gemäß Anspruch 20 der Formel IIIa

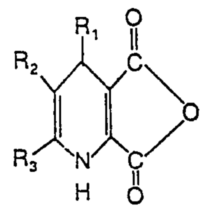

(IIIa)

worin $R_1$, $R_2$ und $R_3$ die in Anspruch 1 angegebene Bedeutung haben.

22. 1,4-Dihydropyridin-2,3-dicarbonsäurederivate gemäß Anspruch 20 der Formel IIIb

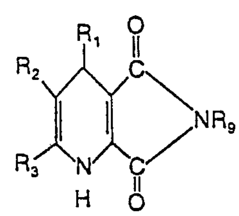

(IIIb)

worin $R_1$, $R_2$, $R_3$ und $R_9$ die in Anspruch 1 angegebene Bedeutung haben.

**Patentansprüche für den Vertragsstaat: AT**

1. Verfahren zur Herstellung von Pyridin-2,3-dicarbonsäurederivaten der Formel I

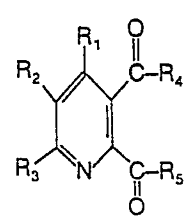

(I)

worin

$R_1$ Wasserstoff, $C_1$–$C_6$-Alkyl, $C_1$–$C_6$-Alkylthio oder $C_1$–$C_6$-Alkoxy, oder durch Hydroxy, Halogen, $C_1$–$C_4$-Alkoxy, Phenyl, Phenoxy, Cyano, Carboxyl oder $C_1$–$C_4$-Alkoxycarbonyl substituiertes $C_1$–$C_6$-Alkyl, $C_1$–$C_6$-Alkylthio oder $C_1$–$C_6$-Alkoxy, oder Phenyl oder Phenoxy, oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Phenoxy bedeutet,

$R_2$ die gleiche Bedeutung hat wie $R_1$ und zusätzlich Fluor, Chlor, Brom bedeutet,

$R_3$ für Wasserstoff steht oder zusammen mit $R_2$ eine Trimethylen- oder Tetramethylengruppe bildet,

$R_4$ und $R_5$ unabhängig voneinander $-OH$, $-NR_2$, $-NHR_6$, $-NR_6T_7$ oder $-OR_8$ sind, wobei

$R_6$ und $R_7$ für $C_1$–$C_6$-Alkyl, $C_5$–$C_6$-Cycloalkyl, Allyl, Methallyl, Propargyl, Phenyl, Benzyl oder durch Halogen, Cyano, $C_1$–$C_4$-Alkyl, $C_1$–$C_4$-Alkoxy, $C_1$–$C_4$-Halogenalkyl oder $C_1$–$C_4$-Cyanoalkyl substituiertes Phenyl oder Benzyl stehen oder $R_6$ und $R_7$ zusammen $C_4$–$C_5$-Alkylen oder 3-Oxapentylen bedeuten,

$R_8$ für $C_1$–$C_6$-Alkyl, $C_5$–$C_6$-Cycloalkyl, Allyl, Methallyl, Propargyl, Phenyl oder Benzyl oder durch $C_1$–$C_4$-Alkyl, $C_1$–$C_4$-Alkoxy oder Halogen substituiertes Phenyl oder Benzyl steht, oder

$R_4$ und $R_5$ zusammen –O– oder $-NR_9-$ bedeuten, wobei

$R_9$ Wasserstoff, $C_1$–$C_6$-Alkyl, Allyl, Methallyl, Propargyl, Phenyl, Benzyl, $C_5$–$C_6$-Cycloalkyl; oder durch $-OH$, $-OR_{12}$, $-SR_{12}$, $-COOH$, $-COOR_8$, $-OCOR_{12}$, $-CONH_2$, $-CONHR_6$, $-CONR_6R_7$, Halogen oder Cyano substituiertes $C_1$–$C_{13}$-Alkyl; oder durch $C_1$–$C_4$-Alkyl, $C_1$–$C_4$-Alkoxy oder Halogen substituiertes Phenyl oder Benzyl und

$R_{12}$ $C_1$–$C_6$-Alkyl, Cyclohexyl oder Phenyl bedeutet,

dadurch gekennzeichnet, daß man ein 1-Amino-1,2,3,4-tetrahydropyridin-2,3-dicarbonsäurederivat der Formel II

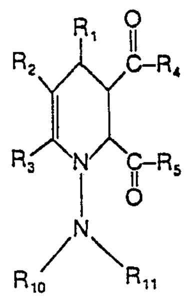

(II)

in welcher

$R_{10}$ und $R_{11}$ unabhängig voneinander $C_1$–$C_6$-Alkyl, $C_5$–$C_6$-Cycloalkyl, Benzyl oder Phenyl oder zusammen $C_4$–$C_5$-Alkylen oder 3-Oxapentylen bedeuten, durch Behandeln mit einer Säure und/oder durch thermische Behandlung bei mindestens 20 °C unter Abspaltung von $R_{10}R_{11}NH$ in ein 1,4-Dihydropyridin-2,3-dicarbonsäurederivat der Formel III

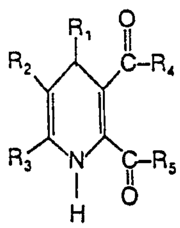

(III)

und anschließend dieses 1,4-Dihydropyridin-2,3-dicarbonsäurederivat der Formel III durch Oxidation in ein Pyridin-2,3-dicarbonsäurederivat umwandelt.

2. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III bei Temperaturen von 20–200 °C durchführt.

3. Verfahren nach Anspruch 2, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III bei 40–150 °C in einem inerten Lösungsmittel durchführt.

4. Verfahren nach Anspruch 3, dadurch gekennzeichnet, daß man als inerte Lösungsmittel Toluol oder Ethanol verwendet.

5. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III in Gegenwart einer organischen Säure aus der Gruppe Ameisensäure, Essigsäure oder Propionsäure durchführt.

6. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III in Gegenwart eines sauren Silikats durchführt.

7. Verfahren nach Anspruch 1, dadurch gekennzeichnet, daß man die Überführung des 1-Amino-1,2,3,4-tetrahydropyridin-2,3-dicarbonsäurederivates der Formel II in das 1,4-Dihydropyridin-2,3-dicarbonsäurederivat der Formel III durch Erhitzen des 1-Amino-1,2,3,4-tetrahydropyridin-2,3-dicarbonsäurederivates der Formel II mit etwa der doppelten Menge Kieselgel in Toluol bei Rückflußtemperatur durchführt.

8. Verfahren zur Herstellung von Pyridin-2,3-dicarbonsäurederivaten der Formel I gemäß Anspruch 1, dadurch gekennzeichnet, daß man ein α,β-ungesättigtes Hydrazon der Formel IV

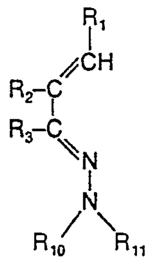

(IV)

in welcher $R_1$, $R_2$, $R_3$, $R_{10}$ und $R_{11}$ die unter Formeln I und II angegebene Bedeutung haben, und ein Ethen-1,2-dicarbonsäurederivat der Formel V

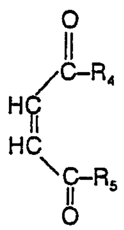

(V),

in welcher $R_4$ und $R_5$ die unter Formel I angegebene Bedeutung haben, in einem inerten Lösungsmittel auf Temperaturen von 40–150 °C erhitzt und das gebildete 1,4-Dihydropyridin-2,3-dicarbonsäurederivat der Formel III anschließend durch Oxidation in ein Pyridin-2,3-dicarbonsäurederivat der Formel I überführt.

9. Verfahren nach Anspruch 8, dadurch gekennzeichnet, daß man als Lösungsmittel Acetonitril oder ein niederes Alkanol verwendet.

10. Verfahren nach Ansprüchen 1 und 8, dadurch gekennzeichnet, daß man zur Überführung des 1,4-Dihydropyridin-2,3-dicarbonsäurederivates der Formel III in ein Pyridin-2,3-dicarbonsäurederivat der Formel I Luft, Sauerstoff, Wasserstoffperoxid, Mangandioxid, Chromsäure, als Oxidationsmittel verwendet.

11. Verfahren nach Anspruch 10, dadurch gekennzeichnet, daß man die Oxidation des 1,4-Dihydropyridin-2,3-dicarbonsäurederivates der Formel II zum Pyridin-2,3-dicarbonsäurederivat der Formel I mit Luft durchführt.

12. Verfahren nach Anspruch 10, dadurch gekennzeichnet, daß man die Oxidation des 1,4-Dihydropyridin-2,3-dicarbonsäurederivates der Formel III zum Pyridin-2,3-dicarbonsäurederivat der Formel I in Gegenwart einer organischen Säure bei 20–200 °C durchführt.

13. Verfahren nach Anspruch 10, dadurch gekennzeichnet, daß man die Oxidation des 1,4-Dihydropyridin-2,3-dicarbonsäurederivates der Formel III zum Pyridin-2,3-dicarbonsäurederivat der Formel I durch Erwärmen mit der im wesentlichen äquimolaren Menge Mangandioxid in Essigsäure bei Temperaturen von 50–100 °C durchführt.

14. Verfahren nach Ansprüchen 1 und 8, dadurch gekennzeichnet, daß man ein 1-Amino-1,2,3,4-tetrahydropyridin-2,3-dicarbonsäurederivat der Formel II bzw. ein Ethen-1,2-dicarbonsäurederivat der Formel V verwendet, in welcher $R_4$ und $R_5$ für einen Rest der Formel verwendet, in welcher $R_4$ und $R_5$ für einen Rest der Formel

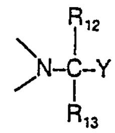

,

stehen, worin Y für $-CN$ oder $-CONH_2$ steht und $R_{12}$ und $R_{13}$ unabhängig voneinander für ein Wasserstoffatom oder lineares oder verzweigtes $C_1$–$C_6$-Alkyl stehen.

15. Verfahren nach Ansprüchen 1 und 8, dadurch gekennzeichnet, daß man ein 1-Amino-1,2,3,4-tetrahydropyridin-2,3-dicarbonsäurederivat der Formel II bzw. ein Ethen-1,2-dicarbonsäurederivat der Formel V verwendet, worin $R_4$ und $R_5$ zusammen für

–O–, 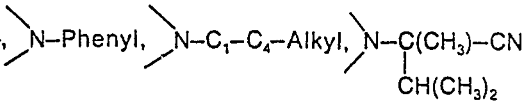

oder 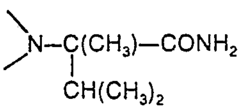

stehen.

16. Verfahren nach Ansprüchen 1 und 8, dadurch gekennzeichnet, daß man Pyridin-2,3-dicarbonsäurederivate der Formel I herstellt, in welchen $R_1$ Wasserstoff oder Methyl, $R_2$ Wasserstoff, $C_1$–$C_6$-Alkyl, Fluor, Chlor, Brom, $C_1$–$C_6$-Alkoxy, $C_1$–$C_4$-Alkylthio, Phenoxy, $C_1$–$C_6$-Hydroxyalkyl, $C_1$–$C_6$-Halogenalkyl, unsubstituiertes oder durch $C_1$–$C_4$-Alkyl, $C_1$–$C_4$-Alkoxy oder Halogen substituiertes Phenyl, $R_3$ Wasserstoff und $R_4$ und $R_5$ einzeln je $-OH$ oder $-OR_8$ oder zusammen –O– oder $-NR_9-$ bedeuten.

17. Verfahren nach Ansprüchen 1 und 8, dadurch gekennzeichnet, daß man Pyridin-2,3-dicarbonsäurederivate der Formel I herstellt in welchen $R_1$ Wasserstoff, $R_2$ $C_1$–$C_6$-Alkyl, $R_3$ Wasserstoff und $R_4$ und $R_5$ einzeln je $-OH$ oder $-OR_8$ oder zusammen eine Gruppe

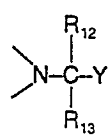

,

bedeuten, wobei $R_8$, $R_{12}$ und $R_{13}$ jeweils die unter Formeln I und II angegebene Bedeutung haben und Y eine Cyano- oder eine Carbamoylgruppe bedeutet.

**Claims for the Contracting States: DE, GB, FR, CH, LI, IT, NL, BE, SE**

1. A process for producing pyridine-2,3-dicarboxylic acid derivatives of formula I

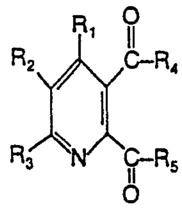

(I)

wherein

$R_1$ is hydrogen, $C_1$–$C_6$alkyl, $C_1$–$C_6$alkylthio or $C_1$–$C_6$alkoxy, or $C_1$–$C_6$alkyl, $C_1$–$C_6$alkylthio or $C_1$–$C_6$alkoxy each substituted by hydroxy, halogen, $C_1$–$C_4$alkoxy, phenyl, phenoxy, cyano, carboxy or by $C_1$–$C_4$alkoxycarbonyl, or phenyl or phenoxy, or phenyl or phenoxy each substituted by halogen, cyano, $C_1$–$C_4$alkyl, $C_1$–$C_4$alkoxy, $C_1$–$C_4$haloalkyl or by $C_1$–$C_4$cyanoalkyl,

$R_2$ has the same meaning as $R_1$ and is additionally fluorine, chlorine or bromine,

$R_3$ is hydrogen or together with $R_2$ forms a trimethylene or tetramethylene group,

$R_4$ and $R_5$ independently of one another are each –OH, $-NH_2$, $-NHR_6$, $-NR_6R_7$ or $-OR_8$ in which

$R_6$ and $R_7$ are $C_1$–$C_6$alkyl, $C_5$–$C_6$cycloalkyl, allyl, methallyl, propargyl, phenyl, benzyl, or phenyl or benzyl each substituted by halogen, cyano, $C_1$–$C_4$alkyl, $C_1$–$C_4$alkoxy, $C_1$–$C_4$haloalkyl or by $C_1$–$C_4$cyanoalkyl, or $R_6$ and $R_7$ together are $C_4$–$C_5$alkylene or 3-oxapentylene,

$R_8$ is $C_1$–$C_6$alkyl, $C_5$–$C_6$cycloalkyl, allyl, methallyl, propargyl, phenyl or benzyl, or phenyl or benzyl each substituted by $C_1$–$C_4$alkyl, $C_1$–$C_4$alkoxy or by halogen, or

$R_4$ and $R_5$ together are –O– or $-NR_9-$ in which

$R_9$ is hydrogen, $C_1$–$C_6$alkyl, allyl, methallyl, propargyl, phenyl, benzyl, $C_5$–$C_6$cycloalkyl; or $C_1$–$C_{13}$alkyl substituted by –OH, $-OR_{12}$, $-SR_{12}$, –COOH, $-COOR_8$, $-OCOR_{12}$, $-CONH_2$, $-CONHR_6$, $-CONR_6R_7$, halogen or by cyano; or phenyl or benzyl each substituted by $C_1$–$C_4$alkyl, $C_{1-4}$alkoxy or by halogen, and

$R_{12}$ is $C_1$–$C_6$alkyl, cyclohexyl or phenyl,

which process comprises converting a 1-amino-1,2,3,4-tetrahydropyridine-2,3-dicarboxylic acid derivative of formula II

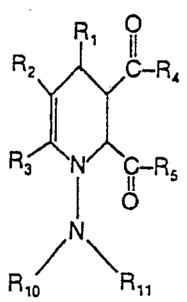

(II)

in which

$R_{10}$ and $R_{11}$ independently of one another are each $C_1$–$C_6$alkyl, $C_5$–$C_6$cycloalkyl, benzyl or phenyl or together are $C_4$–$C_5$-alkylene or 3-oxapentylene, by treatment with an acid and/or by thermal treatment at at least 20 °C with the removal of $R_{10}R_{11}NH$ into a 1,4-dihydropyridine-2,3-dicarboxylic acid derivative of formula III

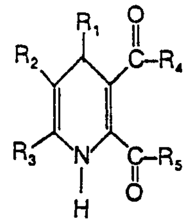

(III)

and subsequently converting this 1,4-dihydropyridine-2,3-dicarboxylic acid derivative of formula III by oxidation into a pyridine-2,3-dicarboxylic acid derivative.

2. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed at temperatures of from 20° to 200 °C.

3. A process according to claim 2, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed at from 40° to 150 °C in an inert solvent.

4. A process according to claim 3, wherein the inert solvent used is toluene or ethanol.

5. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed in the presence of an organic acid from the group formic acid, acetic acid and propionic acid.

6. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed in the presence of an acid silicate.

7. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed by heating the 1-amino-1,2,3,4-tetrahydropyridine-2,3-dicarboxylic acid derivative of formula II with reflux temperature.

8. A process for producing pyridine-2,3-dicarboxylic acid derivatives of formula I according to claim 1, which process comprises heating an α,β-unsaturated hydrazone of formula IV

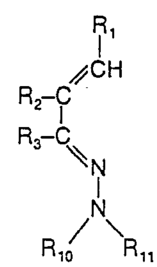

(IV).

in which $R_1$, $R_2$, $R_3$, $R_{10}$ and $R_{11}$ are as defined for formulae I and II, and an ethene-1,2-dicarboxylic acid derivative of formula V

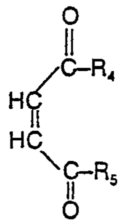

(V),

in which $R_4$ and $R_5$ are as defined for formula I, in an inert solvent at temperatures of from 40° to 150 °C, and subsequently converting the resulting 1,4-dihydropyridine-2,3-dicarboxylic acid derivative of formula III by oxidation into a pyridine-2,3-dicarboxylic acid derivative of formula I.

9. A process according to claim 8, wherein the solvent used is acetonitrile or a lower alkanol.

10. A process according to claims 1 and 8, wherein the oxidising agent used for converting the 1,4-dihydropyridine-2,3-dicarboxylic acid derivative of formula III into a pyridine-2,3-dicarboxylic acid derivative of formula I is air, oxygen, hydrogen peroxide, manganese dioxide or chromic acid.

11. A process according to claim 10, wherein the oxidation of the 1,4-dihydropyridine-2,3-carboxylic acid derivative of formula II to the pyridine-2,3-dicarboxylic acid derivative of formula I is performed with air.

12. A process according to claim 10, wherein the oxidation of the 1,4-dihydropyridine-2,3-dicarboxylic acid derivative of formula III to the pyridine-2,3-dicarboxylic acid derivative of formula I is performed in the presence of an organic acid at from 20° to 200 °C.

13. A process according to claim 10, wherein the oxidation of the 1,4-dihydropyridine-2,3-dicarboxylic acid derivative of formula III to the pyridine-2,3-dicarboxylic acid derivative of formula I is performed by heating with substantially the equimolar amount of manganese dioxide in acetic acid at temperatures of from 50° to 100 °C.

14. A process according to claims 1 and 8, wherein there is used a 1-amino-1,2,3,4-tetrahydropyridine-2,3-dicarboxylic acid derivative of formula II or an ethene-1,2-dicarboxylic acid derivative of formula V in which $R_4$ and $R_5$ are a radical of the formula

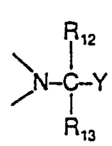

,

wherein Y is $-CN$ or $-CONH_2$, and $R_{12}$ and $R_{13}$ independently of one another are each a hydrogen atom, or linear or branched $C_1$–$C_6$alkyl.

15. A process according to claims 1 and 8, wherein there is used a 1-amino-1,2,3,4-tetrahydropyridine-2,3-dicarboxylic acid derivative of formula II or an ethene-1,2-dicarboxylic acid derivative of formula V in which $R_4$ and $R_5$ together are

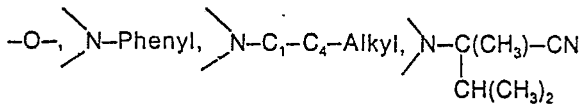

or

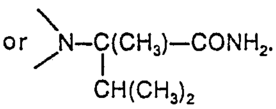

16. A process according to claims 1 and 8, wherein there are produced pyridine-2,3-dicarboxylic acid derivatives of formula I in which $R_1$ is hydrogen or methyl, $R_2$ is hydrogen, $C_1$–$C_6$alkyl, fluorine, chlorine, bromine, $C_1$–$C_6$alkoxy, $C_1$–$C_4$alkylthio, phenoxy, $C_1$–$C_6$hydroxyalkyl, $C_1$–$C_6$haloalkyl, or phenyl unsubstituted or substituted by $C_1$–$C_4$alkyl, $C_1$–$C_4$alkoxy or by halogen, $R_3$ is hydrogen, and $R_4$ and $R_5$ individually are each $-OH$ or $-OR_8$, or together are $-O-$ or $-NR_9-$.

17. A process according to claims 1 and 8, wherein there are produced pyridine-2,3-dicarboxylic acid derivatives of formula I in which $R_1$ is hydrogen, $R_2$ is $C_1$–$C_6$alkyl, $R_3$ is hydrogen, and $R_4$ and $R_5$ individually are each $-OH$ or $-OR_8$, or together are a group

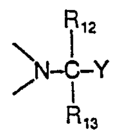

,

in which $R_8$, $R_{12}$ and $R_{13}$ are each as defined for formulae I and II, and Y is a cyano group or a carbamoyl group.

18. 1-Amino-1,2,3,4-tetrahydropyridine-2,3-dicarboxylic acid derivatives of formula II

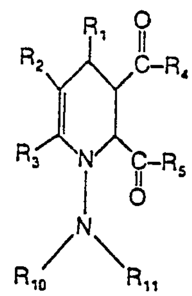

(II)

wherein $R_1$, $R_2$, $R_3$, $R_4$, $R_5$, $R_{10}$ and $R_{11}$ are as defined in claim 1, with the proviso that when $R_1$ is hydrogen and $R_2$ is methyl $R_4$ and $R_5$ are not OH, $OCH_3$ or $OC_2H_5$ or together $-O-$.

19. 1-Amino-1,2,3,4-tetrahydropyridine-2,3-dicarboxylic acid derivatives according to claim 18, of formula IIa

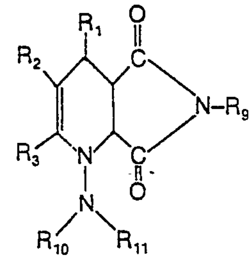

(IIa)

wherein $R_1$, $R_2$, $R_3$, $R_9$, $R_{10}$ and $R_{11}$ are as defined in claim 1.

20. 1,4-Dihydropyridine-2,3-dicarboxylic acid derivatives of formula III

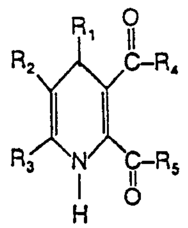

(III)

wherein $R_1$, $R_2$, $R_3$, $R_4$ and $R_5$ are as defined in claim 1.

21. 1,4-Dihydropyridine-2,3-dicarboxylic acid derivatives according to claim 20, of formula IIIa

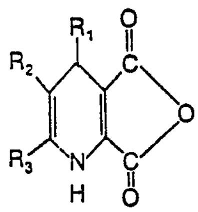

(IIIa)

wherein $R_1$, $R_2$ and $R_3$ are as defined in claim 1.

22. 1,4-Dihydropyridine-2,3-dicarboxylic acid derivatives according to claim 20, of formula IIIb

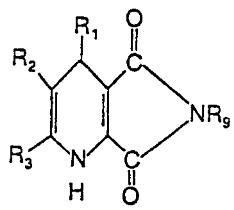

(IIIb)

wherein $R_1$, $R_2$, $R_3$ and $R_9$ are as defined in claim 1.

**Claims for the Contracting State: AT**

1. A process for producing pyridine-2,3-dicarboxylic acid derivatives of formula I

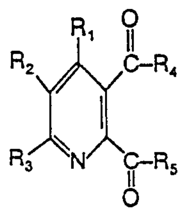

(I)

wherein

$R_1$ is hydrogen, $C_1$–$C_6$alkyl, $C_1$–$C_6$alkylthio or $C_1$–$C_6$alkoxy, or $C_1$–$C_6$alkyl, $C_1$–$C_6$alkylthio or $C_1$–$C_6$alkoxy each substituted by hydroxy, halogen, $C_1$–$C_4$alkoxy, phenyl, phenoxy, cyano, carboxy or by $C_1$–$C_4$alkoxycarbonyl, or phenyl or phenoxy, or phenyl or phenoxy each substituted by halogen, cyano, $C_1$–$C_4$alkyl, $C_1$–$C_4$alkoxy, $C_1$–$C_4$haloalkyl or by $C_1$–$C_4$cyanoalkyl,

$R_2$ has the same meaning as $R_1$ and is additionally fluorine, chlorine or bromine,

$R_3$ is hydrogen or together with $R_2$ forms a trimethylene or tetramethylene group,

$R_4$ and $R_5$ independently of one another are each –OH, –$NH_2$, –$NHR_6$, –$NR_6R_7$ or –$OR_8$ in which

$R_6$ and $R_7$ are $C_1$–$C_6$alkyl, $C_5$–$C_6$cycloalkyl, allyl, methallyl, propargyl, phenyl, benzyl, or phenyl or benzyl each substituted by halogen, cyano, $C_1$–$C_4$alkyl, $C_1$–$C_4$alkoxy, $C_1$–$C_4$haloalkyl or by $C_1$–$C_4$cyanoalkyl, or $R_6$ and $R_7$ together are $C_4$–$C_5$alkylene or 3-oxapentylene,

$R_8$ is $C_1$–$C_6$alkyl, $C_5$–$C_6$cycloalkyl, allyl, methallyl, propargyl, phenyl or benzyl, or phenyl or benzyl each substituted by $C_1$–$C_4$alkyl, $C_1$–$C_4$alkoxy or by halogen, or

$R_4$ and $R_5$ are –O– or –$NR_9$– in which

$R_9$ is hydrogen, $C_1$–$C_6$alkyl, allyl, methallyl, propargyl, phenyl, benzyl, $C_5$–$C_6$cycloalkyl; or $C_1$–$C_{13}$alkyl substituted by –OH, –$OR_{12}$, –$SR_{12}$, –COOH, –$COOR_8$, –$OCOR_{12}$,–$CONH_2$, –$CONHR_6$, –$CONR_6R_7$, halogen or by cyano; or phenyl or benzyl each substituted by $C_1$–$C_4$alkyl, $C_1$–$C_4$alkoxy or by halogen, and

$R_{12}$ is $C_1$–$C_5$alkyl, cyclohexyl or phenyl,

which process comprises converting a 1-amino-1,2,3,4-tetrahydropyridine-2,3-dicarboxylic acid derivative of formula II

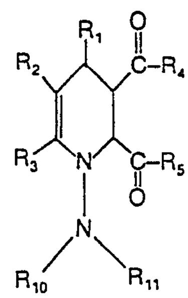

(II)

in which

$R_{10}$ and $R_{11}$ independently of one another are each $C_1$–$C_6$alkyl, $C_5$–$C_6$cycloalkyl, benzyl or phenyl or together are $C_4$–$C_5$alkylene or 3-oxapentylene, by treatment with an acid and/or by thermal treatment at at least 20° with the removal of $R_{10}R_{11}NH$ into a 1,4-dihydropyridine-2,3-dicarboxylic acid derivative of formula III

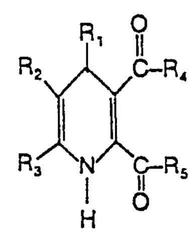

(III)

and subsequently converting this 1,4-dihydropyridine-2,3-dicarboxylic acid derivative of formula III by oxidation into a pyridine-2,3-dicarboxylic acid derivative.

2. A process according to claim 1, wherein the conversion of the 1-amino-1,2,3,4-tetrahydropyridine-2,3-dicarboxylic acid derivative of formula II into the 1,4-dihydropyridine-2,3-carboxylic acid

derivative of formula III is performed at temperatures of from 20° to 200 °C.

3. A process according to claim 2, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed at from 40° to 150 °C in an inert solvent.

4. A process according to claim 3, wherein the inert solvent used is toluene or ethanol.

5. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed in the presence of an organic acid from the group formic acid, acetic acid and propionic acid.

6. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed in the presence of an acid silicate.

7. A process according to claim 1, wherein the conversion of the 1-amino-1,2,3,4-tetrahydropyridine-2,3-dicarboxylic acid derivative of formula II into the 1,4-dihydropyridine-2,3-dicarboxylic acid derivative of formula III is performed by heating the 1-amino-1,2,3,4-tetrahydropyridine-2,3-dicarboxylic acid derivative of formula II with approximately double the amount of silica gel in toluene at reflux temperature.

8. A process for producing pyridine-2,3-dicarboxylic acid derivatives of formula I according to claim 1, which process comprises heating an α,β-unsaturated hydrazone of formula IV

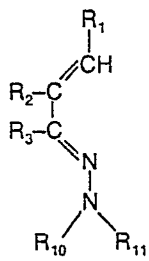

(IV)

in which $R_1$, $R_2$, $R_3$, $R_{10}$ and $R_{11}$ are as defined for formulae I and II, and an ethene-1,2-dicarboxylic acid derivative of formula V

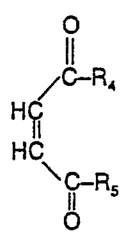

(V)

in which $R_4$ and $R_5$ are as defined for formula I, in an inert solvent at temperatures of from 40° to 150 °C, and subsequently converting the resulting 1,4-dihydropyridine-2,3-dicarboxylic acid derivative of formula III by oxidation into a pyridine-2,3-dicarboxylic acid derivative of formula I.

9. A process according to claim 8, wherein the solvent used is acetonitrile or a lower alkanol.

10. A process according to claims 1 and 8, wherein the oxidising agent used for converting the 1,4-dihydropyridine-2,3-dicarboxylic acid derivative of formula III into a pyridine-2,3-dicarboxylic acid derivative of formula I is air, oxygen, hydrogen peroxide, manganese dioxide or chromic acid.

11. A process according to claim 10, wherein the oxidation of the 1,4-dihydropyridine-2,3-dicarboxylic acid derivative of formula II to the pyridine-2,3-dicarboxylic acid derivative of formula I is performed with air.

12. A process according to claim 10, wherein the oxidation of the 1,4-dihydropyridine-2,3-dicarboxylic acid derivative of formula III to the pyridine-2,3-dicarboxylic acid derivative of formula I is performed in the presence of an organic acid at from 20° to 200 °C.

13. A process according to claim 10, wherein the oxidation of the 1,4-dihydropyridine-2,3-dicarboxylic acid derivative of formula III to the pyridine-2,3-dicarboxylic acid derivative of formula I is performed by heating with substantially the equimolar amount of manganese dioxide in acetic acid at temperatures of from 50° to 100 °C.

14. A process according to claims 1 and 8, wherein there is used a 1-amino-1,2,3,4-tetrahydropyridine-2,3-dicarboxylic acid derivative of formula II or an ethene-1,2-dicarboxylic acid derivative of formula V in which $R_4$ and $R_5$ are a radical of the formula

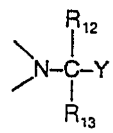

wherein Y is –CN or $-CONH_2$, and $R_{12}$ and $R_{13}$ independently of one another are each a hydrogen atom, or linear or branched $C_1$–$C_6$alkyl.

15. A process according to claims 1 and 8, wherein there is used a 1-amino-1,2,3,4-tetrahydropyridine-2,3-dicarboxylic acid derivative of formula II or an ethene-1,2-dicarboxylic acid derivative of formula V in which $R_4$ and $R_5$ together are

–O–, 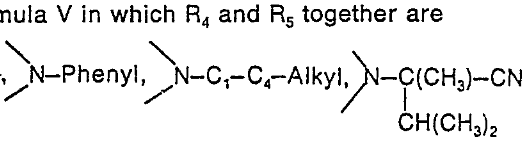

or 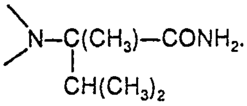

16. A process according to claims 1 and 8, wherein there are produced pyridine-2,3-dicarboxylic acid derivatives of formula I in which $R_1$ is hydrogen or methyl, $R_2$ is hydrogen, $C_1$–$C_6$alkyl, fluorine, chlorine, bromine, $C_1$–$C_6$alkoxy, $C_1$–$C_4$alkylthio, phenoxy, $C_1$–$C_6$hydroxyalkyl, $C_1$–$C_6$haloalkyl, or phenyl unsubstituted or substituted by $C_1$–$C_4$alkyl, $C_1$–$C_4$alkoxy or by halogen, $R_3$ is hydrogen, and $R_4$ and $R_5$ individually are each –OH or $-OR_8$, or together are –O– or $-NR_9-$.

17. A process according to claims 1 and 8, wherein there are produced pyridine-2,3-dicarboxylic acid derivatives of formula I in which $R_1$ is hydrogen, $R_2$ is $C_1$–$C_6$alkyl, $R_3$ is hydrogen, and $R_4$ and $R_5$ individually are each –OH or –$OR_8$, or together are a group

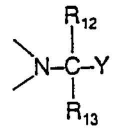

in which $R_8$, $R_{12}$ and $R_{13}$ are each as defined for formulae I and II, and Y is a cyano group or a carbamoyl group.

**Revendications pour les Etats contractants: DE, GB, FR, CH, LI, IT, NL, BE, SE**

1. Procédé de préparation de dérivés d'acide pyridine-2,3-dicarboxyliques de formule I

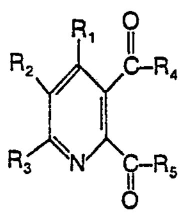

(I)

dans laquelle

$R_1$ est l'hydrogène, un alkyle $C_1$–$C_6$, alkylthio $C_1$–$C_6$ ou alcoxy $C_1$–$C_6$ ou un alkyle $C_1$–$C_6$, alkylthio $C_1$–$C_6$ ou alcoxy $C_1$–$C_6$ substitué par un hydroxy, halogène, alcoxy $C_1$–$C_4$, phényle, phénoxy, cyano, carboxyle ou alcoxycarbonyle $C_1$–$C_4$, ou un phényle ou un phénoxy ou un phényle ou un phénoxy substitués par un halogène, un cyano, un alkyle $C_1$–$C_4$, un alcoxy $C_1$–$C_4$, un halogénoalkyle $C_1$–$C_4$ ou cyanoalkyle $C_1$–$C_4$,

$R_2$ a la même signification que $R_1$ et peut représenter en plus le fluor, le chlore ou le brome,

$R_3$ représente l'hydrogène ou forme avec $R_2$ un groupe triméthylène ou tétraméthylène,

$R_4$ et $R_5$ représentent indépendamment l'un de l'autre –OH, –$NH_2$, –$NHR_6$, $NR_6R_7$ ou –$OR_8$, dans lesquels

$R_6$ et $R_7$ représentent un alkyle $C_1$–$C_6$, un cycloalkyle $C_5$–$C_6$, un allyle, un méthallyle, un propargyle, un phényle, un benzyle ou un benzyle ou un phényle substitué par un halogène, un cyano, un alkyle $C_1$–$C_4$, un alcoxy $C_1$–$C_4$, un halogénoalkyle $C_1$–$C_4$ ou un cyanoalkyle $C_1$–$C_4$ ou $R_6$ et $R_7$ représentent ensemble un alkylène $C_4$–$C_5$ ou un 3-oxapentylène,

$R_8$ représente un alkyle $C_1$–$C_6$, un cycloalkyle $C_5$–$C_6$, un allyle, un méthallyle, un propargyle, un phényle ou un benzyle ou un phényle ou un benzyle substitué par un alkyle $C_1$–$C_4$, un alcoxy $C_1$–$C_4$ ou un halogène, ou

$R_4$ et $R_5$ représentent ensemble –O– ou –$NR_9$–,

$R_9$ représentant l'hydrogène, un alkyle $C_1$–$C_6$, l'allyle, le méthallyle, le propargyle, le phényle, le benzyle, un cycloalkyle $C_5$–$C_6$; ou un alkyle $C_1$–$C_{13}$ substitué par –OH, –$OR_{12}$, –$SR_{12}$, –COOH, –$COOR_8$, –$OCOR_{12}$, –$CONH_2$, –$CONHR_6$, –$CONR_6R_7$, un halogène ou un cyano; ou un phényle ou un benzyle substitué par un alkyle $C_1$–$C_4$, alcoxy $C_1$–$C_4$ ou halogène et

$R_{12}$ représente un alkyle $C_1$–$C_6$, le cyclohexyle ou le phényle,

caractérisé en ce qu'on transforme un dérivé d'acide 1-amino-1,2,3,4-tétrahydropyridine-2,3-dicarboxylique de formule II

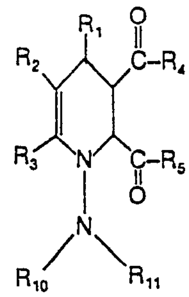

(II)

dans laquelle $R_{10}$ et $R_{11}$ représentent indépendamment l'un de l'autre un alkyle $C_1$–$C_6$, un cycloalkyle $C_5$–$C_6$, le benzyle ou le phényle ou représentent ensemble un alkylène $C_4$–$C_5$ ou un 3-oxapentylène, par traitement avec un acide et/ou par traitement thermique à 20 °C au moins en éliminant $R_{10}R_{11}NH$, en un dérivé d'acide 1,4-dihydropyridine-2,3-dicarboxylique de formule III

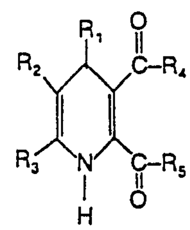

(III)

et ensuite on transforme ce dérivé d'acide 1,4-dihydropyridine-2,3-dicarboxylique de formule III par oxydation en un dérivé d'acide pyridine-2,3-dicarboxylique.

2. Procédé selon la revendication 1, caractérisé en ce que la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III est réalisée à des températures comprises entre 20 et 200 °C.

3. Procédé selon la revendication 2, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III entre 40 et 150 °C dans un solvant inerte.

4. Procédé selon la revendication 3, caractérisé en ce qu'on utilise comme solvant inerte du toluène ou de l'éthanol.

5. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III en présence d'un acide organique du groupe de l'acide formique, de l'acide acétique ou de l'acide propionique.

6. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III en présence d'un silicate acide.

7. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III par chauffage du dérivé d'acide 1-amino-1,2,3,4-tétrahydropyridine-2,3-dicarboxylique de formule II avec environ la quantité double de gel de silice, dans le toluène à la température de reflux.

8. Procédé de préparation de dérivés d'acide pyridine-2,3-dicarboxylique de formule I selon la revendication 1, caractérisé en ce qu'on chauffe une α,β-hydrazone insaturée de formule IV

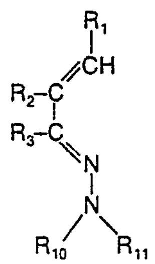

(IV)

dans laquelle $R_1$, $R_2$, $R_3$, $R_{10}$ et $R_{11}$ ont la signification donnée pour les formules I et II, et un dérivé d'acide éthène-1,2-dicarboxylique de formule V,

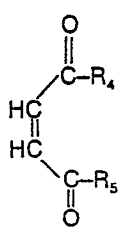

(V)

dans lequel $R_4$ et $R_5$ ont la signification donné pour la formule I, dans un solvant inerte, à des températures de 40 à 150 °C, et le dérivé d'acide 1,4-dihydropyridine-2,3-dicarboxylique de formule III est ensuite transformé par oxydation en un dérivé d'acide pyridine-2,3-dicarboxylique de formule I.

9. Procédé selon la revendication 8, caractérisé en ce qu'on utilise comme solvant de l'acétonitrile ou un alcanol inférieur.

10. Procédé selon les revendications 1 et 8, caractérisé en ce qu'on utilise de l'air, de l'oxygène, du peroxyde d'hydrogène, du dioxyde de manganèse, de l'acide chromique comme agent oxydant pour transformer le dérivé d'acide 1,4-dihydropyridine-2,3-dicarboxylique de formule III en un dérivé d'acide pyridine-2,3-dicarboxylique de formule I.

11. Procédé selon la revendication 10, caractérisé en ce qu'on réalise l'oxydation du dérivé d'acide 1,4-dihydropyridine-2,3-dicarboxylique de formule II en le dérivé d'acide pyridine-2,3-dicarboxylique de formule I avec de l'air.

12. Procédé selon la revendication 10, caractérisé en ce qu'on réalise l'oxydation du dérivé d'acide 1,4-dihydropyridine-2,3-dicarboxylique de formule III en le dérivé d'acide pyridine-2,3-dicarboxylique de formule I en présence d'un acide organique entre 20 et 200 °C.

13. Procédé selon la revendication 10, caractérisé en ce qu'on réalise l'oxydation du dérivé d'acide 1,4-dihydropyridine-2,3-dicarboxylique de formule III en le dérivé d'acide pyridine-2,3-dicarboxylique de formule I par chauffage avec une quantité sensiblement équimolaire de dioxyde de manganèse dans l'acide acétique à des températures comprises entre 50 et 100 °C.

14. Procédé selon les revendications 1 et 8, caractérisé en ce qu'on utilise un dérivé d'acide 1-amino-1,2,3,4-tétrahydropyiridine-2,3-dicarboxylique de formule II ou bien un dérivé d'acide éthène-1,2-dicarboxylique de formule V, dans lequel $R_4$ et $R_5$ représentent un reste de formule

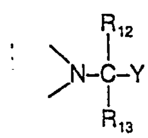

,

dans lequel Y représente –CN ou $-CONH_2$ et $R_{12}$ et $R_{13}$ indépendamment l'un de l'autre représentent un atome d'hydrogène ou un alkyle $C_1$–$C_6$ linéaire ou ramifié.

15. Procédé selon les revendications 1 et 8, caractérisé en ce qu'on utilise un dérivé d'acide 1-amino-1,2,3,4-tétrahydropyridine-2,3-dicarboxylique de formule II ou bien un dérivé d'acide éthène-1,2-dicarboxylique de formule V, dans lequel $R_4$ et $R_5$ représentent ensemble

–O–, 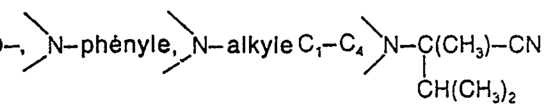

ou 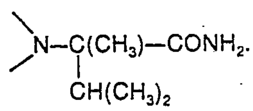

16. Procédé selon les revendications 1 et 8, caractérisé en ce qu'on prépare des dérivés d'acide pyridine-2,3-dicarboxylique de formule I, dans lesquels $R_1$ représente l'hydrogène ou le méthyle, $R_2$ l'hydrogène, un alkyle $C_1$–$C_6$, le fluor, le chlore, le brome, un alcoxy $C_1$–$C_6$, un alkylthio $C_1$–$C_4$, un phénoxy, un hydroxyalkyle $C_1$–$C_6$, un halogénoalkyle $C_1$–$C_6$, un phényle non substitué ou substitué par un alkyle $C_1$–$C_4$, un alcoxy $C_1$–$C_4$ ou un halogène, $R_3$ représente l'hydrogène et $R_4$ et $R_5$ représentent chacun –OH ou $-OR_8$ ou ensemble –O– ou $-NR_9-$.

17. Procédé selon les revendications 1 et 8, caractérisé en ce qu'on prépare des dérivés d'acide pyridine-2,3-dicarboxylique de formule I dans lesquels $R_1$ est l'hydrogène, $R_2$ est un alkyle $C_1$–$C_6$, $R_3$ est l'hydrogène et $R_4$ et $R_5$ représentent chacun –OH ou $-OR_8$ ou ensemble un groupe

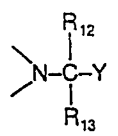,

dans lesquels $R_8$, $R_{12}$ et $R_{13}$ ont chacun les significations données pour les formules I et II et Y représente un groupe cyano ou carbamoyle.

18. Dérivés d'acide 1-amino-1,2,3,4-tétrahydropyridine de formule II

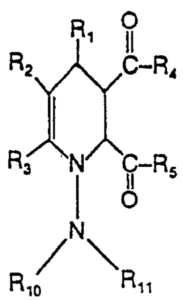

(II)

dans laquelle $R_1$, $R_2$, $R_3$, $R_4$, $R_5$, $R_{10}$ et $R_{11}$ ont la signification indiquée dans la revendication 1, à la condition que $R_4$ et $R_5$ ne représentent pas OH, $OCH_3$, $OC_2H_5$ ou, ensemble, –O–, quand $R_1$ représente l'hydrogène et $R_2$ le méthyle.

19. Dérivés d'acide 1-amino-1,2,3,4-tétrahydropyridine-2,3-dicarboxylique selon la revendication 18 de formule IIa

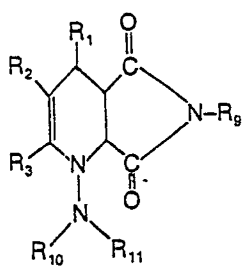

(IIa)

dans laquelle $R_1$, $R_2$, $R_3$, $R_9$, $R_{10}$ et $R_{11}$ ont la signification donnée dans la revendication 1.

20. Dérivés d'acide 1,4-dihydropyridine-2,3-dicarboxylique de formule III

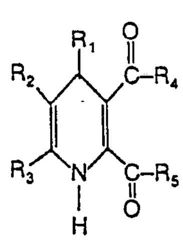

(III)

dans laquelle $R_1$, $R_2$, $R_3$, $R_4$ et $R_5$ ont la signification donnée dans la revendication 1.

21. Dérivés d'acide 1,4-dihydropyridine selon la revendication 20 de formule IIIa

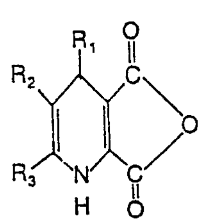

(IIIa)

dans laquelle $R_1$, $R_2$ et $R_3$ ont la signification donnée dans la revendication 1.

22. Dérivés d'acide 1,4-dihydropyridine-2,3-dicarboxylique selon la revendication 20 de formule IIIb

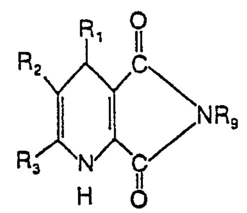

(IIIb)

dans laquelle $R_1$, $R_2$, $R_3$ et $R_9$ ont la signification donnée dans la revendication 1.

**Revendications pour l'Etat contractant: AT**

1. Procédé de préparation de dérivés d'acide pyridine-2,3-dicarboxyliques de formule I

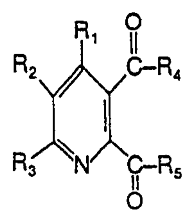

(I)

dans laquelle

$R_1$ est l'hydrogène, un alkyle $C_1$–$C_6$, alkylthio $C_1$–$C_6$ ou alcoxy $C_1$–$C_6$ ou un alkyle $C_1$–$C_6$, alkylthio $C_1$–$C_6$ ou alcoxy $C_1$–$C_6$ substitué par un hydroxy, halogène, alcoxy $C_1$–$C_4$, phényle, phénoxy, cyano, carboxyle ou alcoxycarbonyle $C_1$–$C_4$, ou un phényle ou un phénoxy ou un phényle ou un phénoxy substitué par un halogène, un cyano, un alkyle $C_1$–$C_4$, un alcoxy $C_1$–$C_4$, un halogénoalkyle $C_1$–$C_4$ ou cyanoalkyle $C_1$–$C_4$,

$R_2$ a la même signification que $R_1$ et peut représenter en plus le fluor, le chlore ou le brome,

$R_3$ représente l'hydrogène ou forme avec $R_2$ un groupe triméthylène ou tétraméthylène,

$R_4$ et $R_5$ représentent indépendamment l'un de l'autre –OH, $-NH_2$, $-NHR_6$, $-NR_6R_7$ ou $-OR_8$, dans lesquels,

$R_6$ et $R_7$ représentent un alkyle $C_1$–$C_6$, un cycloalkyle $C_5$–$C_6$, un allyle, un méthallyle, un propargyle, un phényle, un benzyle ou un benzyle ou un phényle substitué par un halogène, un cyano, un alkyle $C_1$–$C_4$, un alcoxy $C_1$–$C_4$, un halogénoalkyle $C_1$–$C_4$ ou un cyanoalkyle $C_1$–$C_4$ ou $R_6$ et $R_7$ représentent ensemble un alkylène $C_4$–$C_5$ ou un 3-oxapentylène,

$R_8$ représente un alkyle $C_1$–$C_6$, un cycloalkyle $C_5$–$C_6$, un allyle, un méthallyle, un propargyle, un phényle ou un benzyle ou un phényle ou un benzyle substitués par un alkyle $C_1$–$C_4$, un alcoxy $C_1$–$C_4$ ou un halogène, ou

$R_4$ et $R_5$ représentent ensemble –O– ou $-NR_9-$,

$R_9$ représentant l'hydrogène, un alkyle $C_1$–$C_6$, l'allyle, le méthallyle, le propargyle, le phényle, le benzyle, un cycloalkyle $C_5$–$C_6$, ou un alkyle $C_1$–$C_{13}$ substitué par –OH, $-OR_{12}$, $-SR_{12}$, –COOH, $-COOR_8$, $-OCOR_{12}$, $-CONH_2$, $-CONHR_6$, $-CONR_6R_7$, un halo-

gène ou un cyano; ou un phényle ou un benzyle substitué par un alkyle $C_1$–$C_4$, alcoxy $C_1$–$C_4$ ou halogène et

$R_{12}$ représente un alkyle $C_1$–$C_6$, le cyclohexyle ou le phényle,

caractérisé en ce qu'on transforme un dérivé d'acide 1-amino-1,2,3,4-tétrahydropyridine-2,3-dicarboxylique de formule II

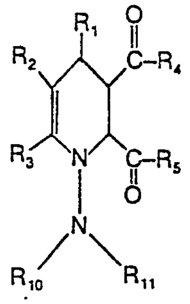

(II)

dans laquelle $R_{10}$ et $R_{11}$ représentent indépendamment l'un de l'autre un alkyle $C_1$–$C_6$, un cycloalkyle $C_5$–$C_6$, la benzyle ou le phényle ou représentent ensemble un alkylène $C_4$–$C_5$ ou un 3-oxapentylène, par traitement avec un acide et/ou par traitement thermique à 20 °C au moins en éliminant $R_{10}R_{11}NH$, en un dérivé d'acide 1,4-dihydropyridine-2,3-dicarboxylique de formule III

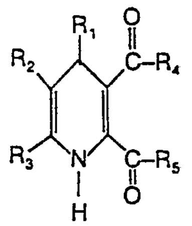

(III)

et ensuite on transforme ce dérivé d'acide 1,4-dihydropyridine-2,3-dicarboxylique de formule III par oxydation en un dérivé d'acide pyridine-2,3-dicarboxylique.

2. Procédé selon la revendication 1, caractérisé en ce que la transformation du dérivé d'acide 1-amio-1,2,3,4-tétrahydropyridine-2,3-dicarboxylique de formule II en le dérivé d'acide 1,4-dihydropyridine-2,3-dicarboxylique de formule III est réalisée à des températures comprises entre 20 et 200 °C.

3. Procédé selon la revendication 2, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III entre 40 et 150 °C dans un solvant inerte.

4. Procédé selon la revendication 3, caractérisé en ce qu'on utilise comme solvant inerte du toluène ou de l'éthanol.

5. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III en présence d'un acide organique du groupe de l'acide formique, de l'acide acétique ou de l'acide propionique.

6. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III en présence d'un silicate acide.

7. Procédé selon la revendication 1, caractérisé en ce qu'on réalise la transformation du dérivé d'acide 1-amino-1,2,3,4-tétrahydropyridine-2,3-dicarboxylique de formule II en le dérivé d'acide 1,4-dihydropyridine-2,3-dicarboxylique de formule III par chauffage du dérivé d'acide 1-amino-1,2,3,4-tétrahydropyridine-2,3-dicarboxylique de formule II avec environ la quantité double de gel de silice, dans le toluène à la température de reflux.

8. Procédé de préparation de dérivés d'acide pyridine-2,3-dicarboxylique de formule I selon la revendication 1, caractérisé en ce qu'on chauffe une α,β-hydrazone insaturée de formule IV

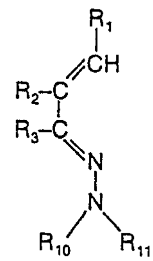

(IV),

dans laquelle $R_1$, $R_2$, $R_3$, $R_{10}$ et $R_{11}$ ont la signification donnée pour les formules I et II, et un dérivé d'acide éthène-1,2-dicarboxylique de formule V

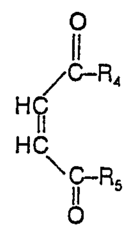

(V),

dans lequel $R_4$ et $R_5$ ont la signification donnée pour la formule I, dans un solvant inerte, à des températures de 40 à 150 °C, et le dérivé d'acide 1,4-dihydropyridine-2,3-dicarboxylique de formule III est ensuite transformé par oxydation en un dérivé d'acide pyridine-2,3-dicarboxylique de formule I.

9. Procédé selon la revendication 8, caractérisé en ce qu'on utilise comme solvant de l'acétonitrile ou un alcanol inférieur.

10. Procédé selon les revendications 1 et 8, caractérisé en ce qu'on utilise de l'air, de l'oxygène, du peroxyde d'hydrogène, du dioxyde de manganèse, de l'acide chromique comme agent oxydant pour transformer le dérivé d'acide 1,4-dihydropyridine-2,3-dicarboxylique de formule III en un dérivé d'acide pyridine-2,3-dicarboxylique de formule I.

11. Procédé selon la revendication 10, caractérisé en ce qu'on réalise l'oxydation du dérivé d'acide 1,4-dihydropyridine-2,3-dicarboxylique de

formule II en le dérivé d'acide pyridine-2,3-dicarboxylique de formule I avec de l'air.

12. Procédé selon la revendication 10, caractérisé en ce qu'on réalise l'oxydation du dérivé d'acide 1,4-dihydropyridine-2,3-dicarboxylique de formule III en le dérivé d'acide pyridine-2,3-dicarboxylique de formule I en présence d'un acide organique entre 20 et 200 °C.

13. Procédé selon la revendication 10, caractérisé en ce qu'on réalise l'oxydation du dérivé d'acide 1,4-dihydropyridine-2,3-dicarboxylique de formule III en le dérivé d'acide pyridine-2,3-dicarboxylique de formule I par chauffage avec une quantité sensiblement équimolaire de dioxyde de manganèse dans l'acide acétique à des températures comprises entre 50 et 100 °C.

14. Procédé selon les revendications 1 et 8, caractérisé en ce qu'on utilise un dérivé d'acide 1-amino-1,2,3,4-tétrahydropyridine-2,3-dicarboxylique de formule II ou bien un dérivé d'acide éthène-1,2-dicarboxylique de formule V, dans lequel $R_4$ et $R_5$ représentent un reste de formule

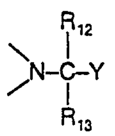,

dans lequel Y représente –CN ou –$CONH_2$ et $R_{12}$ et $R_{13}$ indépendamment l'un de l'autre représentent un atome d'hydrogène ou un alkyle $C_1$–$C_6$ linéaire ou ramifié.

15. Procédé selon les revendications 1 et 8, caractérisé en ce qu'on utilise un dérivé d'acide 1-amino-1,2,3,4-tétrahydropyridine-2,3-dicarboxylique de formule II ou bien un dérivé d'acide éthène-1,2-dicarboxylique de formule V, dans lequel $R_4$ et $R_5$ représentent ensemble

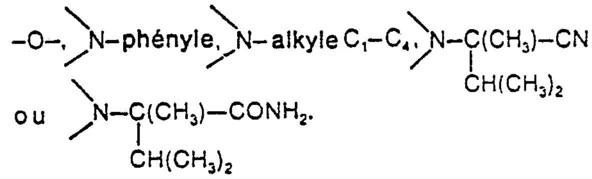

16. Procédé selon les revendications 1 et 8, caractérisé en ce qu'on prépare des dérivés d'acide pyridine-2,3-dicarboxylique de formule I, dans lesquels $R_1$ représente l'hydrogène ou le méthyle, $R_2$ l'hydrogène un alkyle $C_1$–$C_6$, le fluor, le chlore, le brome, un alcoxy $C_1$–$C_6$, un alkylthio $C_1$–$C_4$, un phénoxy, un hydroxyalkyle $C_1$–$C_6$, un halogénoalkyle $C_1$–$C_6$, un phényle non substitué ou substitué par un alkyle $C_1$–$C_4$, un alcoxy $C_1$–$C_4$ ou un halogène, $R_3$ représente l'hydrogène et $R_4$ et $R_5$ représentent chacun –OH ou –$OR_8$ ou ensemble –O– ou –$NR_9$–.

17. Procédé selon les revendications 1 et 8, caractérisé en ce qu'on prépare des dérivés d'acide pyridine-2,3-dicarboxylique de formule I dans lesquels $R_1$ est l'hydrogène, $R_2$ est un alkyle $C_1$–$C_6$, $R_3$ est l'hydrogène et $R_4$ et $R_5$ représentent chacun –OH ou –$OR_8$ ou ensemble un groupe

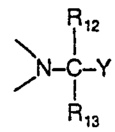,

dans lesquels $R_8$, $R_{12}$ et $R_{13}$ ont chacun les significations données pour les formules I et II et Y représente un groupe cyano ou carbamoyle.